# EUROPEAN PATENT APPLICATION

(11) **EP 1 616 880 A1**
(43) Date of publication of application: **18.01.2006**
(21) Application number: 04720260.1
(22) Date of filing: 12.03.2004
(51) Int. Cl.: C07K 16/28, A61K 39/395, A61P 7/00, A61P 7/04, G01N 33/15, G01N 33/50

(54) **LIGAND HAVING AGONISTIC ACTIVITY TO MUTATED RECEPTOR**

(30) Priority: 13.03.2003 JP 2003067832
(71) Applicant: CHUGAI SEIYAKU KABUSHIKI KAISHA, Tokyo, 115-8543 (JP)
(72) Inventor: TSUCHIYA, Masayuki, CHUGAI SEIYAKU K.K., Gotenba-shi, Shizuoka 4120038 (JP); HIRATA, Yuichi, CHUGAI SEIYAKU K.K., Niihari-gun, Ibaraki 3004101 (JP)
(74) Representative: Vossius & Partner
(86) International application number: PCT/JP2004/003334
(87) International publication number: WO 2004/081048

(57) **Abstract**

The present inventors used antibody engineering techniques to prepare functional antibodies that correspond to individual mutations in causative genes of diseases, and discovered that such antibodies enable the treatment of the diseases. Specifically, the inventors succeeded in preparing ligands, particularly minibodies, which have agonistic activity to receptors that have almost completely lost responsiveness to their natural ligands because of gene mutations (for example, a thrombopoietin (TPO) receptor whose reactivity to TPO has been markedly impaired), and which can transduce signals by interacting with these mutant receptors at levels comparable to normal.

SEQUENCE LISTING

<110> CHUGAI SEIYAKU KABUSHIKI KAISHA

<120> Ligand having agonistic activity to mutated receptor

<130> C1-A0303P

<150> JP 2003-067832
<151> 2003-03-13

<160> 20

<170> PatentIn version 3.1

<210> 1
<211> 1924
<212> DNA
<213> Homo sapiens

<220>
<221> CDS
<222> (11)..(1918)
<223>

<400> 1
gaattccacc atg ccc tcc tgg gcc ctc ttc atg gtc acc tcc tgc ctc 49
ctc ctg gcc cct caa aac ctg gcc caa gtc agc agc caa gat gtc tcc 97
ttg ctg gca tca gac tca gag ccc ctg aag tgt ttc tcc cga aca ttt 145
gag gac ctc act tgc ttc tgg gat gag gaa gag gca gcg ccc agt ggg 193
aca tac cag ctg ctg tat gcc tac ccg cgg gag aag ccc cgt gct tgc 241
ccc ctg agt tcc cag agc atg ccc cac ttt gga acc cga tac gtg tgc 289
cag ttt cca gac cag gag gaa gtg cgt ctc ttc ttt ccg ctg cac ctc 337
tgg gtg aag aat gtg ttc cta aac cag act cgg act cag cga gtc ctc 385
ttt gtg gac agt gta ggc ctg ccg gct ccc ccc agt atc atc aag gcc 433
atg ggt ggg agc cag cca ggg gaa ctt cag atc agc tgg gag gag cca 481
gct cca gaa atc agt gat ttc ctg agg tac gaa ctc cgc tat ggc ccc 529
aga gat ccc aag aac tcc act ggt ccc acg gtc ata cag ctg att gcc 577
aca gaa acc tgc tgc cct gct ctg cag aga cct cac tca gcc tct gct 625
ctg gac cag tct cca tgt gct cag ccc aca atg ccc tgg caa gat gga 673
cca aag cag acc tcc cca agt aga gaa gct tca gct ctg aca gca gag 721
ggt gga agc tgc ctc atc tca gga ctc cag cct ggc aac tcc tac tgg 769
ctg cag ctg cgc agc gaa cct gat ggg atc tcc ctc ggt ggc tcc tgg 817
gga tcc tgg tcc ctc cct gtg act gtg gac ctg cct gga gat gca gtg 865
gca ctt gga ctg caa tgc ttt acc ttg gac ctg aag aat gtt acc tgt 913
caa tgg cag caa cag gac cat gct agc tcc caa ggc ttc ttc tac cac 961
agc agg gca cgg tgc tgc ccc aga gac agg tac ccc atc tgg gag aac 1009
tgc gaa gag gaa gag aaa aca aat cca gga cta cag acc cca cag ttc 1057
tct cgc tgc cac ttc aag tca cga aat gac agc att att cac atc ctt 1105
gtg gag gtg acc aca gcc ccg ggt act gtt cac agc tac ctg ggc tcc 1153
cct ttc tgg atc cac cag gct gtg cgc ctc ccc acc cca aac ttg cac 1201
tgg agg gag atc tcc agt ggg cat ctg gaa ttg gag tgg cag cac cca 1249
tcg tcc tgg gca gcc caa gag acc tgt tat caa ctc cga tac aca gga 1297
gaa ggc cat cag gac tgg aag gtg ctg gag ccg cct ctc ggg gcc cga 1345
gga ggg acc ctg gag ctg cgc ccg cga tct cgc tac cgt tta cag ctg 1393
cgc gcc agg ctc aac ggc ccc acc tac caa ggt ccc tgg agc tcg tgg 1441
tcg gac cca act agg gtg gag acc gcc acc gag acc gcc tgg atc tcc 1489
ttg gtg acc gct ctg cat cta gtg ctg ggc ctc agc gcc gtc ctg ggc 1537
ctg ctg ctg ctg agg tgg cag ttt cct gca cac tac agg aga ctg agg 1585
cat gcc ctg tgg ccc tca ctt cca gac ctg cac cgg gtc cta ggc cag     1633
tac ctt agg gac act gca gcc ctg agc ccg ccc aag gcc aca gtc tca     1681
gat acc tgt gaa gaa gtg gaa ccc agc ctc ctt gaa atc ctc ccc aag     1729
tcc tca gag agg act cct ttg ccc ctg tgt tcc tcc cag gcc cag atg     1777
gac tac cga aga ttg cag cct tct tgc ctg ggg acc atg ccc ctg tct     1825
gtg tgc cca ccc atg gct gag tca ggg tcc tgc tgt acc acc cac att     1873
gcc aac cat tcc tac cta cca cta agc tat tgg cag cag cct tga 1918
gtcgac 1924

<210> 2
<211> 635
<212> PRT
<213> Homo sapiens

<400> 2

<210>  3
<211>  1924
<212>  DNA
<213>  Homo sapiens

<220>
<221>  CDS
<222>  (11)..(1918)
<223>

<400>  3
gaattccacc atg ccc tcc tgg gcc ctc ttc atg gtc acc tcc tgc ctc        49
           Met Pro Ser Trp Ala Leu Phe Met Val Thr Ser Cys Leu
           1               5                   10

ctc ctg gcc cct caa aac ctg gcc caa gtc agc agc caa gat gtc tcc        97
Leu Leu Ala Pro Gln Asn Leu Ala Gln Val Ser Ser Gln Asp Val Ser
    15                  20                  25

ttg ctg gca tca gac tca gag ccc ctg aag tgt ttc tcc cga aca ttt       145
Leu Leu Ala Ser Asp Ser Glu Pro Leu Lys Cys Phe Ser Arg Thr Phe
30                  35                  40                  45

gag gac ctc act tgc ttc tgg gat gag gaa gag gca gcg ccc agt ggg 193
Glu Asp Leu Thr Cys Phe Trp Asp Glu Glu Glu Ala Ala Pro Ser Gly
                50                  55                  60

aca tac cag ctg ctg tat gcc tac ccg cgg gag aag ccc cgt gct tgc 241
Thr Tyr Gln Leu Leu Tyr Ala Tyr Pro Arg Glu Lys Pro Arg Ala Cys
            65                  70                  75

ccc ctg agt tcc cag agc atg ccc cac ttt gga acc cga tac gtg tgc 289
Pro Leu Ser Ser Gln Ser Met Pro His Phe Gly Thr Arg Tyr Val Cys
        80                  85                  90

cag ttt cca gac cag gag gaa gtg cct ctc ttc ttt ccg ctg cac ctc 337
Gln Phe Pro Asp Gln Glu Glu Val Pro Leu Phe Phe Pro Leu His Leu
    95                  100                 105

tgg gtg aag aat gtg ttc cta aac cag act cgg act cag cga gtc ctc 385
Trp Val Lys Asn Val Phe Leu Asn Gln Thr Arg Thr Gln Arg Val Leu
110                 115                 120                 125

ttt gtg gac agt gta ggc ctg ccg gct ccc ccc agt atc atc aag gcc 433
Phe Val Asp Ser Val Gly Leu Pro Ala Pro Pro Ser Ile Ile Lys Ala
                130                 135                 140

atg ggt ggg agc cag cca ggg gaa ctt cag atc agc tgg gag gag cca 481

Met Gly Gly Ser Gln Pro Gly Glu Leu Gln Ile Ser Trp Glu Glu Pro
            145                 150                 155

gct cca gaa atc agt gat ttc ctg agg tac gaa ctc cgc tat ggc ccc 529
Ala Pro Glu Ile Ser Asp Phe Leu Arg Tyr Glu Leu Arg Tyr Gly Pro
        160                 165                 170

aga gat ccc aag aac tcc act ggt ccc acg gtc ata cag ctg att gcc 577
Arg Asp Pro Lys Asn Ser Thr Gly Pro Thr Val Ile Gln Leu Ile Ala
    175                 180                 185

aca gaa acc tgc tgc cct gct ctg cag aga cct cac tca gcc tct gct 625
Thr Glu Thr Cys Cys Pro Ala Leu Gln Arg Pro His Ser Ala Ser Ala
190                 195                 200                 205

ctg gac cag tct cca tgt gct cag ccc aca atg ccc tgg caa gat gga 673
Leu Asp Gln Ser Pro Cys Ala Gln Pro Thr Met Pro Trp Gln Asp Gly
                210                 215                 220

cca aag cag acc tcc cca agt aga gaa gct tca gct ctg aca gca gag 721
Pro Lys Gln Thr Ser Pro Ser Arg Glu Ala Ser Ala Leu Thr Ala Glu
            225                 230                 235

ggt gga agc tgc ctc atc tca gga ctc cag cct ggc aac tcc tac tgg 769
Gly Gly Ser Cys Leu Ile Ser Gly Leu Gln Pro Gly Asn Ser Tyr Trp
        240                 245                 250

ctg cag ctg cgc agc gaa cct gat ggg atc tcc ctc ggt ggc tcc tgg 817
Leu Gln Leu Arg Ser Glu Pro Asp Gly Ile Ser Leu Gly Gly Ser Trp
    255                 260                 265

gga tcc tgg tcc ctc cct gtg act gtg gac ctg cct gga gat gca gtg 865
Gly Ser Trp Ser Leu Pro Val Thr Val Asp Leu Pro Gly Asp Ala Val
270                 275                 280                 285

gca ctt gga ctg caa tgc ttt acc ttg gac ctg aag aat gtt acc tgt 913
Ala Leu Gly Leu Gln Cys Phe Thr Leu Asp Leu Lys Asn Val Thr Cys
                290                 295                 300

caa tgg cag caa cag gac cat gct agc tcc caa ggc ttc ttc tac cac 961
Gln Trp Gln Gln Gln Asp His Ala Ser Ser Gln Gly Phe Phe Tyr His
            305                 310                 315

agc agg gca cgg tgc tgc ccc aga gac agg tac ccc atc tgg gag aac 1009
Ser Arg Ala Arg Cys Cys Pro Arg Asp Arg Tyr Pro Ile Trp Glu Asn
        320                 325                 330

tgc gaa gag gaa gag aaa aca aat cca gga cta cag acc cca cag ttc 1057
Cys Glu Glu Glu Glu Lys Thr Asn Pro Gly Leu Gln Thr Pro Gln Phe
    335                 340                 345

tct cgc tgc cac ttc aag tca cga aat gac agc att att cac atc ctt 1105

Ser Arg Cys His Phe Lys Ser Arg Asn Asp Ser Ile Ile His Ile Leu
350                 355                 360                 365

gtg gag gtg acc aca gcc ccg ggt act gtt cac agc tac ctg ggc tcc     1153
Val Glu Val Thr Thr Ala Pro Gly Thr Val His Ser Tyr Leu Gly Ser
                370                 375                 380

cct ttc tgg atc cac cag gct gtg cgc ctc ccc acc cca aac ttg cac     1201
Pro Phe Trp Ile His Gln Ala Val Arg Leu Pro Thr Pro Asn Leu His
            385                 390                 395

tgg agg gag atc tcc agt ggg cat ctg gaa ttg gag tgg cag cac cca     1249
Trp Arg Glu Ile Ser Ser Gly His Leu Glu Leu Glu Trp Gln His Pro
        400                 405                 410

tcg tcc tgg gca gcc caa gag acc tgt tat caa ctc cga tac aca gga     1297
Ser Ser Trp Ala Ala Gln Glu Thr Cys Tyr Gln Leu Arg Tyr Thr Gly
    415                 420                 425

gaa ggc cat cag gac tgg aag gtg ctg gag ccg cct ctc ggg gcc cga     1345
Glu Gly His Gln Asp Trp Lys Val Leu Glu Pro Pro Leu Gly Ala Arg
430                 435                 440                 445

gga ggg acc ctg gag ctg cgc ccg cga tct cgc tac cgt tta cag ctg     1393
Gly Gly Thr Leu Glu Leu Arg Pro Arg Ser Arg Tyr Arg Leu Gln Leu
                450                 455                 460

cgc gcc agg ctc aac ggc ccc acc tac caa ggt ccc tgg agc tcg tgg 1441
Arg Ala Arg Leu Asn Gly Pro Thr Tyr Gln Gly Pro Trp Ser Ser Trp
            465                 470                 475

tcg gac cca act agg gtg gag acc gcc acc gag acc gcc tgg atc tcc 1489
Ser Asp Pro Thr Arg Val Glu Thr Ala Thr Glu Thr Ala Trp Ile Ser
        480                 485                 490

ttg gtg acc gct ctg cat cta gtg ctg ggc ctc agc gcc gtc ctg ggc 1537
Leu Val Thr Ala Leu His Leu Val Leu Gly Leu Ser Ala Val Leu Gly
    495                 500                 505

ctg ctg ctg ctg agg tgg cag ttt cct gca cac tac agg aga ctg agg 1585
Leu Leu Leu Leu Arg Trp Gln Phe Pro Ala His Tyr Arg Arg Leu Arg
510                 515                 520                 525

cat gcc ctg tgg ccc tca ctt cca gac ctg cac cgg gtc cta ggc cag 1633
His Ala Leu Trp Pro Ser Leu Pro Asp Leu His Arg Val Leu Gly Gln
                530                 535                 540

tac ctt agg gac act gca gcc ctg agc ccg ccc aag gcc aca gtc tca 1681
Tyr Leu Arg Asp Thr Ala Ala Leu Ser Pro Pro Lys Ala Thr Val Ser
            545                 550                 555

gat acc tgt gaa gaa gtg gaa ccc agc ctc ctt gaa atc ctc ccc aag 1729

Asp Thr Cys Glu Glu Val Glu Pro Ser Leu Leu Glu Ile Leu Pro Lys

        560                 565                 570

tcc tca gag agg act cct ttg ccc ctg tgt tcc tcc cag gcc cag atg 1777

Ser Ser Glu Arg Thr Pro Leu Pro Leu Cys Ser Ser Gln Ala Gln Met

    575                 580                 585

gac tac cga aga ttg cag cct tct tgc ctg ggg acc atg ccc ctg tct 1825

Asp Tyr Arg Arg Leu Gln Pro Ser Cys Leu Gly Thr Met Pro Leu Ser

590                 595                 600                 605

gtg tgc cca ccc atg gct gag tca ggg tcc tgc tgt acc acc cac att 1873

Val Cys Pro Pro Met Ala Glu Ser Gly Ser Cys Cys Thr Thr His Ile

                610                 615                 620

gcc aac cat tcc tac cta cca cta agc tat tgg cag cag cct tga 1918

Ala Asn His Ser Tyr Leu Pro Leu Ser Tyr Trp Gln Gln Pro

            625                 630                 635

gtcgac 1924

<210> 4

<211> 635

<212> PRT

<213> Homo sapiens

<400> 4

Met Pro Ser Trp Ala Leu Phe Met Val Thr Ser Cys Leu Leu Leu Ala

1               5                   10                  15

Pro Gln Asn Leu Ala Gln Val Ser Ser Gln Asp Val Ser Leu Leu Ala

            20                  25                  30

Ser Asp Ser Glu Pro Leu Lys Cys Phe Ser Arg Thr Phe Glu Asp Leu

        35                  40                  45

Thr Cys Phe Trp Asp Glu Glu Glu Ala Ala Pro Ser Gly Thr Tyr Gln

    50                  55                  60

Leu Leu Tyr Ala Tyr Pro Arg Glu Lys Pro Arg Ala Cys Pro Leu Ser

65                  70                  75                  80

Ser Gln Ser Met Pro His Phe Gly Thr Arg Tyr Val Cys Gln Phe Pro

                85                  90                  95

Asp Gln Glu Glu Val Pro Leu Phe Phe Pro Leu His Leu Trp Val Lys

            100                 105                 110

Asn Val Phe Leu Asn Gln Thr Arg Thr Gln Arg Val Leu Phe Val Asp

        115                 120                 125

Ser Val Gly Leu Pro Ala Pro Pro Ser Ile Ile Lys Ala Met Gly Gly
    130                 135                 140
Ser Gln Pro Gly Glu Leu Gln Ile Ser Trp Glu Glu Pro Ala Pro Glu
145                 150                 155                 160
Ile Ser Asp Phe Leu Arg Tyr Glu Leu Arg Tyr Gly Pro Arg Asp Pro
                165                 170                 175
Lys Asn Ser Thr Gly Pro Thr Val Ile Gln Leu Ile Ala Thr Glu Thr
            180                 185                 190
Cys Cys Pro Ala Leu Gln Arg Pro His Ser Ala Ser Ala Leu Asp Gln
        195                 200                 205
Ser Pro Cys Ala Gln Pro Thr Met Pro Trp Gln Asp Gly Pro Lys Gln
    210                 215                 220
Thr Ser Pro Ser Arg Glu Ala Ser Ala Leu Thr Ala Glu Gly Gly Ser
225                 230                 235                 240
Cys Leu Ile Ser Gly Leu Gln Pro Gly Asn Ser Tyr Trp Leu Gln Leu
                245                 250                 255
Arg Ser Glu Pro Asp Gly Ile Ser Leu Gly Gly Ser Trp Gly Ser Trp
            260                 265                 270

Ser Leu Pro Val Thr Val Asp Leu Pro Gly Asp Ala Val Ala Leu Gly
        275                 280                 285
Leu Gln Cys Phe Thr Leu Asp Leu Lys Asn Val Thr Cys Gln Trp Gln
    290                 295                 300
Gln Gln Asp His Ala Ser Ser Gln Gly Phe Phe Tyr His Ser Arg Ala
305                 310                 315                 320
Arg Cys Cys Pro Arg Asp Arg Tyr Pro Ile Trp Glu Asn Cys Glu Glu
                325                 330                 335
Glu Glu Lys Thr Asn Pro Gly Leu Gln Thr Pro Gln Phe Ser Arg Cys
            340                 345                 350
His Phe Lys Ser Arg Asn Asp Ser Ile Ile His Ile Leu Val Glu Val
        355                 360                 365
Thr Thr Ala Pro Gly Thr Val His Ser Tyr Leu Gly Ser Pro Phe Trp
    370                 375                 380
Ile His Gln Ala Val Arg Leu Pro Thr Pro Asn Leu His Trp Arg Glu
385                 390                 395                 400
Ile Ser Ser Gly His Leu Glu Leu Glu Trp Gln His Pro Ser Ser Trp

                405                 410                 415

Ala Ala Gln Glu Thr Cys Tyr Gln Leu Arg Tyr Thr Gly Glu Gly His

            420                 425                 430

Gln Asp Trp Lys Val Leu Glu Pro Pro Leu Gly Ala Arg Gly Gly Thr

        435                 440                 445

Leu Glu Leu Arg Pro Arg Ser Arg Tyr Arg Leu Gln Leu Arg Ala Arg

    450                 455                 460

Leu Asn Gly Pro Thr Tyr Gln Gly Pro Trp Ser Ser Trp Ser Asp Pro

465                 470                 475                 480

Thr Arg Val Glu Thr Ala Thr Glu Thr Ala Trp Ile Ser Leu Val Thr

                485                 490                 495

Ala Leu His Leu Val Leu Gly Leu Ser Ala Val Leu Gly Leu Leu Leu

            500                 505                 510

Leu Arg Trp Gln Phe Pro Ala His Tyr Arg Arg Leu Arg His Ala Leu

        515                 520                 525

Trp Pro Ser Leu Pro Asp Leu His Arg Val Leu Gly Gln Tyr Leu Arg

    530                 535                 540

Asp Thr Ala Ala Leu Ser Pro Pro Lys Ala Thr Val Ser Asp Thr Cys
545                 550                 555                 560

Glu Glu Val Glu Pro Ser Leu Leu Glu Ile Leu Pro Lys Ser Ser Glu
                565                 570                 575

Arg Thr Pro Leu Pro Leu Cys Ser Ser Gln Ala Gln Met Asp Tyr Arg
            580                 585                 590

Arg Leu Gln Pro Ser Cys Leu Gly Thr Met Pro Leu Ser Val Cys Pro
        595                 600                 605

Pro Met Ala Glu Ser Gly Ser Cys Cys Thr Thr His Ile Ala Asn His
    610                 615                 620

Ser Tyr Leu Pro Leu Ser Tyr Trp Gln Gln Pro
625                 630                 635

<210>  5
<211>  1506
<212>  DNA
<213>  Homo sapiens

<220>
<221>  CDS

<222>  (1)..(1506)

<223>

<400>  5

atg gta agc gct att gtt tta tat gtg ctt ttg gcg gcg gcg gcg cat      48

Met Val Ser Ala Ile Val Leu Tyr Val Leu Leu Ala Ala Ala Ala His

1               5                   10                  15

tct gcc ttt gcg gat ctg cat caa gat gtc tcc ttg ctg gca tca gac      96

Ser Ala Phe Ala Asp Leu His Gln Asp Val Ser Leu Leu Ala Ser Asp

            20                  25                  30

tca gag ccc ctg aag tgt ttc tcc cga aca ttt gag gac ctc act tgc      144

Ser Glu Pro Leu Lys Cys Phe Ser Arg Thr Phe Glu Asp Leu Thr Cys

        35                  40                  45

ttc tgg gat gag gaa gag gca gcg ccc agt ggg aca tac cag ctg ctg      192

Phe Trp Asp Glu Glu Glu Ala Ala Pro Ser Gly Thr Tyr Gln Leu Leu

    50                  55                  60

tat gcc tac ccg cgg gag aag ccc cgt gct tgc ccc ctg agt tcc cag      240

Tyr Ala Tyr Pro Arg Glu Lys Pro Arg Ala Cys Pro Leu Ser Ser Gln

65                  70                  75                  80

agc atg ccc cac ttt gga acc cga tac gtg tgc cag ttt cca gac cag      288

Ser Met Pro His Phe Gly Thr Arg Tyr Val Cys Gln Phe Pro Asp Gln

                85                  90                  95

gag gaa gtg cgt ctc ttc ttt ccg ctg cac ctc tgg gtg aag aat gtg 336
Glu Glu Val Arg Leu Phe Phe Pro Leu His Leu Trp Val Lys Asn Val
            100                 105                 110

ttc cta aac cag act cgg act cag cga gtc ctc ttt gtg gac agt gta 384
Phe Leu Asn Gln Thr Arg Thr Gln Arg Val Leu Phe Val Asp Ser Val
        115                 120                 125

ggc ctg ccg gct ccc ccc agt atc atc aag gcc atg ggt ggg agc cag 432
Gly Leu Pro Ala Pro Pro Ser Ile Ile Lys Ala Met Gly Gly Ser Gln
    130                 135                 140

cca ggg gaa ctt cag atc agc tgg gag gag cca gct cca gaa atc agt 480
Pro Gly Glu Leu Gln Ile Ser Trp Glu Glu Pro Ala Pro Glu Ile Ser
145                 150                 155                 160

gat ttc ctg agg tac gaa ctc cgc tat ggc ccc aga gat ccc aag aac 528
Asp Phe Leu Arg Tyr Glu Leu Arg Tyr Gly Pro Arg Asp Pro Lys Asn
                165                 170                 175

tcc act ggt ccc acg gtc ata cag ctg att gcc aca gaa acc tgc tgc 576
Ser Thr Gly Pro Thr Val Ile Gln Leu Ile Ala Thr Glu Thr Cys Cys
            180                 185                 190

cct gct ctg cag aga cct cac tca gcc tct gct ctg gac cag tct cca 624
Pro Ala Leu Gln Arg Pro His Ser Ala Ser Ala Leu Asp Gln Ser Pro
        195                 200                 205

tgt gct cag ccc aca atg ccc tgg caa gat gga cca aag cag acc tcc 672
Cys Ala Gln Pro Thr Met Pro Trp Gln Asp Gly Pro Lys Gln Thr Ser
    210                 215                 220

cca agt aga gaa gct tca gct ctg aca gca gag ggt gga agc tgc ctc 720
Pro Ser Arg Glu Ala Ser Ala Leu Thr Ala Glu Gly Gly Ser Cys Leu
225                 230                 235                 240

atc tca gga ctc cag cct ggc aac tcc tac tgg ctg cag ctg cgc agc 768
Ile Ser Gly Leu Gln Pro Gly Asn Ser Tyr Trp Leu Gln Leu Arg Ser
                245                 250                 255

gaa cct gat ggg atc tcc ctc ggt ggc tcc tgg gga tcc tgg tcc ctc 816
Glu Pro Asp Gly Ile Ser Leu Gly Gly Ser Trp Gly Ser Trp Ser Leu
            260                 265                 270

cct gtg act gtg gac ctg cct gga gat gca gtg gca ctt gga ctg caa 864
Pro Val Thr Val Asp Leu Pro Gly Asp Ala Val Ala Leu Gly Leu Gln
        275                 280                 285

tgc ttt acc ttg gac ctg aag aat gtt acc tgt caa tgg cag caa cag 912
Cys Phe Thr Leu Asp Leu Lys Asn Val Thr Cys Gln Trp Gln Gln Gln

    290                 295                 300

gac cat gct agc tcc caa ggc ttc ttc tac cac agc agg gca cgg tgc 960
Asp His Ala Ser Ser Gln Gly Phe Phe Tyr His Ser Arg Ala Arg Cys
305                 310                 315                 320

tgc ccc aga gac agg tac ccc atc tgg gag aac tgc gaa gag gaa gag 1008
Cys Pro Arg Asp Arg Tyr Pro Ile Trp Glu Asn Cys Glu Glu Glu Glu
                325                 330                 335

aaa aca aat cca gga cta cag acc cca cag ttc tct cgc tgc cac ttc 1056
Lys Thr Asn Pro Gly Leu Gln Thr Pro Gln Phe Ser Arg Cys His Phe
            340                 345                 350

aag tca cga aat gac agc att att cac atc ctt gtg gag gtg acc aca 1104
Lys Ser Arg Asn Asp Ser Ile Ile His Ile Leu Val Glu Val Thr Thr
        355                 360                 365

gcc ccg ggt act gtt cac agc tac ctg ggc tcc cct ttc tgg atc cac 1152
Ala Pro Gly Thr Val His Ser Tyr Leu Gly Ser Pro Phe Trp Ile His
    370                 375                 380

cag gct gtg cgc ctc ccc acc cca aac ttg cac tgg agg gag atc tcc 1200
Gln Ala Val Arg Leu Pro Thr Pro Asn Leu His Trp Arg Glu Ile Ser
385                 390                 395                 400

agt ggg cat ctg gaa ttg gag tgg cag cac cca tcg tcc tgg gca gcc 1248
Ser Gly His Leu Glu Leu Glu Trp Gln His Pro Ser Ser Trp Ala Ala
                405                 410                 415

caa gag acc tgt tat caa ctc cga tac aca gga gaa ggc cat cag gac 1296
Gln Glu Thr Cys Tyr Gln Leu Arg Tyr Thr Gly Glu Gly His Gln Asp
            420                 425                 430

tgg aag gtg ctg gag ccg cct ctc ggg gcc cga gga ggg acc ctg gag 1344
Trp Lys Val Leu Glu Pro Pro Leu Gly Ala Arg Gly Gly Thr Leu Glu
        435                 440                 445

ctg cgc ccg cga tct cgc tac cgt tta cag ctg cgc gcc agg ctc aac 1392
Leu Arg Pro Arg Ser Arg Tyr Arg Leu Gln Leu Arg Ala Arg Leu Asn
    450                 455                 460

ggc ccc acc tac caa ggt ccc tgg agc tcg tgg tcg gac cca act agg 1440
Gly Pro Thr Tyr Gln Gly Pro Trp Ser Ser Trp Ser Asp Pro Thr Arg
465                 470                 475                 480

gtg gag acc gcc acc gag acc gcc tgg gtc gac gga tcc gac tac aag 1488
Val Glu Thr Ala Thr Glu Thr Ala Trp Val Asp Gly Ser Asp Tyr Lys
                485                 490                 495

gac gac gat gac aag tga 1506
Asp Asp Asp Asp Lys

            500

<210> 6

<211> 501

<212> PRT

<213> Homo sapiens

<400> 6

Met Val Ser Ala Ile Val Leu Tyr Val Leu Leu Ala Ala Ala Ala His

1               5                   10                  15

Ser Ala Phe Ala Asp Leu His Gln Asp Val Ser Leu Leu Ala Ser Asp

            20                  25                  30

Ser Glu Pro Leu Lys Cys Phe Ser Arg Thr Phe Glu Asp Leu Thr Cys

        35                  40                  45

Phe Trp Asp Glu Glu Glu Ala Ala Pro Ser Gly Thr Tyr Gln Leu Leu

    50                  55                  60

Tyr Ala Tyr Pro Arg Glu Lys Pro Arg Ala Cys Pro Leu Ser Ser Gln

65                  70                  75                  80

Ser Met Pro His Phe Gly Thr Arg Tyr Val Cys Gln Phe Pro Asp Gln

                85                  90                  95

Glu Glu Val Arg Leu Phe Phe Pro Leu His Leu Trp Val Lys Asn Val
            100                 105                 110
Phe Leu Asn Gln Thr Arg Thr Gln Arg Val Leu Phe Val Asp Ser Val
        115                 120                 125
Gly Leu Pro Ala Pro Pro Ser Ile Ile Lys Ala Met Gly Gly Ser Gln
    130                 135                 140
Pro Gly Glu Leu Gln Ile Ser Trp Glu Glu Pro Ala Pro Glu Ile Ser
145                 150                 155                 160
Asp Phe Leu Arg Tyr Glu Leu Arg Tyr Gly Pro Arg Asp Pro Lys Asn
                165                 170                 175
Ser Thr Gly Pro Thr Val Ile Gln Leu Ile Ala Thr Glu Thr Cys Cys
            180                 185                 190
Pro Ala Leu Gln Arg Pro His Ser Ala Ser Ala Leu Asp Gln Ser Pro
        195                 200                 205
Cys Ala Gln Pro Thr Met Pro Trp Gln Asp Gly Pro Lys Gln Thr Ser
    210                 215                 220
Pro Ser Arg Glu Ala Ser Ala Leu Thr Ala Glu Gly Gly Ser Cys Leu

225                 230                 235                 240

Ile Ser Gly Leu Gln Pro Gly Asn Ser Tyr Trp Leu Gln Leu Arg Ser

                245                 250                 255

Glu Pro Asp Gly Ile Ser Leu Gly Gly Ser Trp Gly Ser Trp Ser Leu

            260                 265                 270

Pro Val Thr Val Asp Leu Pro Gly Asp Ala Val Ala Leu Gly Leu Gln

        275                 280                 285

Cys Phe Thr Leu Asp Leu Lys Asn Val Thr Cys Gln Trp Gln Gln Gln

    290                 295                 300

Asp His Ala Ser Ser Gln Gly Phe Phe Tyr His Ser Arg Ala Arg Cys

305                 310                 315                 320

Cys Pro Arg Asp Arg Tyr Pro Ile Trp Glu Asn Cys Glu Glu Glu Glu

                325                 330                 335

Lys Thr Asn Pro Gly Leu Gln Thr Pro Gln Phe Ser Arg Cys His Phe

            340                 345                 350

Lys Ser Arg Asn Asp Ser Ile Ile His Ile Leu Val Glu Val Thr Thr

        355                 360                 365

Ala Pro Gly Thr Val His Ser Tyr Leu Gly Ser Pro Phe Trp Ile His
    370                 375                 380
Gln Ala Val Arg Leu Pro Thr Pro Asn Leu His Trp Arg Glu Ile Ser
385                 390                 395                 400
Ser Gly His Leu Glu Leu Glu Trp Gln His Pro Ser Ser Trp Ala Ala
                405                 410                 415
Gln Glu Thr Cys Tyr Gln Leu Arg Tyr Thr Gly Glu Gly His Gln Asp
            420                 425                 430
Trp Lys Val Leu Glu Pro Pro Leu Gly Ala Arg Gly Gly Thr Leu Glu
        435                 440                 445
Leu Arg Pro Arg Ser Arg Tyr Arg Leu Gln Leu Arg Ala Arg Leu Asn
    450                 455                 460
Gly Pro Thr Tyr Gln Gly Pro Trp Ser Ser Trp Ser Asp Pro Thr Arg
465                 470                 475                 480
Val Glu Thr Ala Thr Glu Thr Ala Trp Val Asp Gly Ser Asp Tyr Lys
                485                 490                 495
Asp Asp Asp Asp Lys
            500

<210> 7

<211> 768

<212> DNA

<213> Mus musculus

<220>

<221> CDS

<222> (1)..(768)

<223>

<400> 7

atg aga gtg ctg att cct ttg tgg ctg ttc aca gcc ttt cct ggt acc 48

Met Arg Val Leu Ile Pro Leu Trp Leu Phe Thr Ala Phe Pro Gly Thr

1               5                   10                  15

ctg tct gat gtg cag ctg cag gag tcg gga cct ggc ctg gtg aaa cct 96

Leu Ser Asp Val Gln Leu Gln Glu Ser Gly Pro Gly Leu Val Lys Pro

            20                  25                  30

tct cag tct ctg tcc ctc acc tgc act gtc act ggc tac tca atc acc 144

Ser Gln Ser Leu Ser Leu Thr Cys Thr Val Thr Gly Tyr Ser Ile Thr

        35                  40                  45

agt gat tat gcc tgg agc tgg atc cga cag tta cca gga aac aaa ctg 192

Ser Asp Tyr Ala Trp Ser Trp Ile Arg Gln Leu Pro Gly Asn Lys Leu
    50                  55                  60

gag tgg atg ggc tac ata acg tac agt ggt tac tct atc tac aat cca 240
Glu Trp Met Gly Tyr Ile Thr Tyr Ser Gly Tyr Ser Ile Tyr Asn Pro
65                  70                  75                  80

tct ctc aaa agt cga atc tct atc agt cga gac aca tcc aag aac cag 288
Ser Leu Lys Ser Arg Ile Ser Ile Ser Arg Asp Thr Ser Lys Asn Gln
                85                  90                  95

ttg ttc ctg cag tta aat tct gtg acc act gag gac aca gcc aca tat 336
Leu Phe Leu Gln Leu Asn Ser Val Thr Thr Glu Asp Thr Ala Thr Tyr
            100                 105                 110

tac tgt gtg ggc ggg tat gac aat atg gac tat tgg ggt caa gga acc 384
Tyr Cys Val Gly Gly Tyr Asp Asn Met Asp Tyr Trp Gly Gln Gly Thr
        115                 120                 125

tca gtc acc gtc tcc tca ggt gga ggc gga tcg caa att gtt ctc acc 432
Ser Val Thr Val Ser Ser Gly Gly Gly Gly Ser Gln Ile Val Leu Thr
    130                 135                 140

cag tct cca gca atc atg tct gca tct cct ggg gag aag gtc acc ttg 480
Gln Ser Pro Ala Ile Met Ser Ala Ser Pro Gly Glu Lys Val Thr Leu
145                 150                 155                 160

acc tgc agt gcc agc tca agt gta agt tcc agc cac tta tac tgg tat 528
Thr Cys Ser Ala Ser Ser Ser Val Ser Ser Ser His Leu Tyr Trp Tyr
                165                 170                 175

cag cag aag cca ggg tcc tcc ccc aaa ctc tgg att tat agc aca tcc 576
Gln Gln Lys Pro Gly Ser Ser Pro Lys Leu Trp Ile Tyr Ser Thr Ser
            180                 185                 190

aac ctg gct tct gga gtc cct gct cgc ttc agt ggc agt ggg tct ggg 624
Asn Leu Ala Ser Gly Val Pro Ala Arg Phe Ser Gly Ser Gly Ser Gly
        195                 200                 205

acc tcc tat tct ctc aca atc agc aac atg gag act gaa gat gct gcc 672
Thr Ser Tyr Ser Leu Thr Ile Ser Asn Met Glu Thr Glu Asp Ala Ala
    210                 215                 220

tct tat ttc tgc cat cag tgg agt agt tac cca tgg acg ttc ggt ggg 720
Ser Tyr Phe Cys His Gln Trp Ser Ser Tyr Pro Trp Thr Phe Gly Gly
225                 230                 235                 240

ggc acc aag ctg gaa atc aaa gac tac aag gat gac gac gat aag tga 768
Gly Thr Lys Leu Glu Ile Lys Asp Tyr Lys Asp Asp Asp Asp Lys
                245                 250                 255

<210> 8

<211> 255

<212> PRT

<213> Mus musculus

<400> 8

Met Arg Val Leu Ile Pro Leu Trp Leu Phe Thr Ala Phe Pro Gly Thr

1               5                   10                  15

Leu Ser Asp Val Gln Leu Gln Glu Ser Gly Pro Gly Leu Val Lys Pro

            20                  25                  30

Ser Gln Ser Leu Ser Leu Thr Cys Thr Val Thr Gly Tyr Ser Ile Thr

        35                  40                  45

Ser Asp Tyr Ala Trp Ser Trp Ile Arg Gln Leu Pro Gly Asn Lys Leu

    50                  55                  60

Glu Trp Met Gly Tyr Ile Thr Tyr Ser Gly Tyr Ser Ile Tyr Asn Pro

65                  70                  75                  80

Ser Leu Lys Ser Arg Ile Ser Ile Ser Arg Asp Thr Ser Lys Asn Gln

                85                  90                  95

Leu Phe Leu Gln Leu Asn Ser Val Thr Thr Glu Asp Thr Ala Thr Tyr

            100                 105                 110

Tyr Cys Val Gly Gly Tyr Asp Asn Met Asp Tyr Trp Gly Gln Gly Thr
        115                 120                 125

Ser Val Thr Val Ser Ser Gly Gly Gly Gly Ser Gln Ile Val Leu Thr
    130                 135                 140

Gln Ser Pro Ala Ile Met Ser Ala Ser Pro Gly Glu Lys Val Thr Leu
145                 150                 155                 160

Thr Cys Ser Ala Ser Ser Ser Val Ser Ser Ser His Leu Tyr Trp Tyr
                165                 170                 175

Gln Gln Lys Pro Gly Ser Ser Pro Lys Leu Trp Ile Tyr Ser Thr Ser
            180                 185                 190

Asn Leu Ala Ser Gly Val Pro Ala Arg Phe Ser Gly Ser Gly Ser Gly
        195                 200                 205

Thr Ser Tyr Ser Leu Thr Ile Ser Asn Met Glu Thr Glu Asp Ala Ala
    210                 215                 220

Ser Tyr Phe Cys His Gln Trp Ser Ser Tyr Pro Trp Thr Phe Gly Gly
225                 230                 235                 240

Gly Thr Lys Leu Glu Ile Lys Asp Tyr Lys Asp Asp Asp Asp Lys

                245                 250                 255

<210> 9
<211> 1924
<212> DNA
<213> Homo sapiens

<220>
<221> CDS
<222> (11)..(1918)
<223>

<400> 9
gaattccacc atg ccc tcc tgg gcc ctc ttc atg gtc acc tcc tgc ctc 49
Met Pro Ser Trp Ala Leu Phe Met Val Thr Ser Cys Leu
1               5                   10

ctc ctg gcc cct caa aac ctg gcc caa gtc agc agc caa gat gtc tcc 97
Leu Leu Ala Pro Gln Asn Leu Ala Gln Val Ser Ser Gln Asp Val Ser
    15                  20                  25

ttg ctg gca tca gac tca gag ccc ctg aag tgt ttc tcc cga aca ttt 145
Leu Leu Ala Ser Asp Ser Glu Pro Leu Lys Cys Phe Ser Arg Thr Phe
30                  35                  40                  45

gag gac ctc act tgc ttc tgg gat gag gaa gag gca gcg ccc agt ggg 193
Glu Asp Leu Thr Cys Phe Trp Asp Glu Glu Glu Ala Ala Pro Ser Gly
                50                  55                  60

aca tac cag ctg ctg tat gcc tac ccg cgg gag aag ccc cgt gct tgc 241
Thr Tyr Gln Leu Leu Tyr Ala Tyr Pro Arg Glu Lys Pro Arg Ala Cys
            65                  70                  75

ccc ctg agt tcc cag agc atg ccc cac ttt gga acc cga tac gtg tgc 289
Pro Leu Ser Ser Gln Ser Met Pro His Phe Gly Thr Arg Tyr Val Cys
        80                  85                  90

cag ttt cca gac cag gag gaa gtg cgt ctc ttc ttt ccg ctg cac ctc 337
Gln Phe Pro Asp Gln Glu Glu Val Arg Leu Phe Phe Pro Leu His Leu
    95                  100                 105

tgg gtg aag aat gtg ttc cta aac cag act cgg act cag cga gtc ctc 385
Trp Val Lys Asn Val Phe Leu Asn Gln Thr Arg Thr Gln Arg Val Leu
110                 115                 120                 125

ttt gtg gac agt gta ggc ctg ccg gct ccc ccc agt atc atc aag gcc 433
Phe Val Asp Ser Val Gly Leu Pro Ala Pro Pro Ser Ile Ile Lys Ala
                130                 135                 140

atg ggt ggg agc cag cca ggg gaa ctt cag atc agc tgg gag gag cca 481
Met Gly Gly Ser Gln Pro Gly Glu Leu Gln Ile Ser Trp Glu Glu Pro

            145                 150                 155

gct cca gaa atc agt gat ttc ctg agg tac gaa ctc cgc tat ggc ccc 529
Ala Pro Glu Ile Ser Asp Phe Leu Arg Tyr Glu Leu Arg Tyr Gly Pro
        160                 165                 170

aga gat ccc aag aac tcc act ggt ccc acg gtc ata cag ctg att gcc 577
Arg Asp Pro Lys Asn Ser Thr Gly Pro Thr Val Ile Gln Leu Ile Ala
    175                 180                 185

aca gaa acc tgc tgc cct gct ctg cag aga cct cac tca gcc tct gct 625
Thr Glu Thr Cys Cys Pro Ala Leu Gln Arg Pro His Ser Ala Ser Ala
190                 195                 200                 205

ctg gac cag tct cca tgt gct cag ccc aca atg ccc tgg caa gat gga 673
Leu Asp Gln Ser Pro Cys Ala Gln Pro Thr Met Pro Trp Gln Asp Gly
                210                 215                 220

cca aag cag acc tcc cca agt aga gaa gct tca gct ctg aca gca gag 721
Pro Lys Gln Thr Ser Pro Ser Arg Glu Ala Ser Ala Leu Thr Ala Glu
            225                 230                 235

ggt gga agc tgc ctc atc tca gga ctc cag cct ggc aac tcc tac tgg 769
Gly Gly Ser Cys Leu Ile Ser Gly Leu Gln Pro Gly Asn Ser Tyr Trp
        240                 245                 250

ctg cag ctg tgc agc gaa cct gat ggg atc tcc ctc ggt ggc tcc tgg 817
Leu Gln Leu Cys Ser Glu Pro Asp Gly Ile Ser Leu Gly Gly Ser Trp
    255                 260                 265

gga tcc tgg tcc ctc cct gtg act gtg gac ctg cct gga gat gca gtg 865
Gly Ser Trp Ser Leu Pro Val Thr Val Asp Leu Pro Gly Asp Ala Val
270                 275                 280                 285

gca ctt gga ctg caa tgc ttt acc ttg gac ctg aag aat gtt acc tgt 913
Ala Leu Gly Leu Gln Cys Phe Thr Leu Asp Leu Lys Asn Val Thr Cys
                290                 295                 300

caa tgg cag caa cag gac cat gct agc tcc caa ggc ttc ttc tac cac 961
Gln Trp Gln Gln Gln Asp His Ala Ser Ser Gln Gly Phe Phe Tyr His
            305                 310                 315

agc agg gca cgg tgc tgc ccc aga gac agg tac ccc atc tgg gag aac 1009
Ser Arg Ala Arg Cys Cys Pro Arg Asp Arg Tyr Pro Ile Trp Glu Asn
        320                 325                 330

tgc gaa gag gaa gag aaa aca aat cca gga cta cag acc cca cag ttc 1057
Cys Glu Glu Glu Glu Lys Thr Asn Pro Gly Leu Gln Thr Pro Gln Phe
    335                 340                 345

tct cgc tgc cac ttc aag tca cga aat gac agc att att cac atc ctt 1105
Ser Arg Cys His Phe Lys Ser Arg Asn Asp Ser Ile Ile His Ile Leu

350                 355                 360                 365

gtg gag gtg acc aca gcc ccg ggt act gtt cac agc tac ctg ggc tcc 1153
Val Glu Val Thr Thr Ala Pro Gly Thr Val His Ser Tyr Leu Gly Ser
                370                 375                 380

cct ttc tgg atc cac cag gct gtg cgc ctc ccc acc cca aac ttg cac 1201
Pro Phe Trp Ile His Gln Ala Val Arg Leu Pro Thr Pro Asn Leu His
            385                 390                 395

tgg agg gag atc tcc agt ggg cat ctg gaa ttg gag tgg cag cac cca 1249
Trp Arg Glu Ile Ser Ser Gly His Leu Glu Leu Glu Trp Gln His Pro
        400                 405                 410

tcg tcc tgg gca gcc caa gag acc tgt tat caa ctc cga tac aca gga 1297
Ser Ser Trp Ala Ala Gln Glu Thr Cys Tyr Gln Leu Arg Tyr Thr Gly
    415                 420                 425

gaa ggc cat cag gac tgg aag gtg ctg gag ccg cct ctc ggg gcc cga 1345
Glu Gly His Gln Asp Trp Lys Val Leu Glu Pro Pro Leu Gly Ala Arg
430                 435                 440                 445

gga ggg acc ctg gag ctg cgc ccg cga tct cgc tac cgt tta cag ctg 1393
Gly Gly Thr Leu Glu Leu Arg Pro Arg Ser Arg Tyr Arg Leu Gln Leu
                450                 455                 460

cgc gcc agg ctc aac ggc ccc acc tac caa ggt ccc tgg agc tcg tgg 1441
Arg Ala Arg Leu Asn Gly Pro Thr Tyr Gln Gly Pro Trp Ser Ser Trp
            465                 470                 475

tcg gac cca act agg gtg gag acc gcc acc gag acc gcc tgg atc tcc 1489
Ser Asp Pro Thr Arg Val Glu Thr Ala Thr Glu Thr Ala Trp Ile Ser
        480                 485                 490

ttg gtg acc gct ctg cat cta gtg ctg ggc ctc agc gcc gtc ctg ggc 1537
Leu Val Thr Ala Leu His Leu Val Leu Gly Leu Ser Ala Val Leu Gly
    495                 500                 505

ctg ctg ctg ctg agg tgg cag ttt cct gca cac tac agg aga ctg agg 1585
Leu Leu Leu Leu Arg Trp Gln Phe Pro Ala His Tyr Arg Arg Leu Arg
510                 515                 520                 525

cat gcc ctg tgg ccc tca ctt cca gac ctg cac cgg gtc cta ggc cag 1633
His Ala Leu Trp Pro Ser Leu Pro Asp Leu His Arg Val Leu Gly Gln
                530                 535                 540

tac ctt agg gac act gca gcc ctg agc ccg ccc aag gcc aca gtc tca 1681
Tyr Leu Arg Asp Thr Ala Ala Leu Ser Pro Pro Lys Ala Thr Val Ser
            545                 550                 555

gat acc tgt gaa gaa gtg gaa ccc agc ctc ctt gaa atc ctc ccc aag 1729
Asp Thr Cys Glu Glu Val Glu Pro Ser Leu Leu Glu Ile Leu Pro Lys

560                 565                 570

tcc tca gag agg act cct ttg ccc ctg tgt tcc tcc cag gcc cag atg      1777
Ser Ser Glu Arg Thr Pro Leu Pro Leu Cys Ser Ser Gln Ala Gln Met
    575                 580                 585

gac tac cga aga ttg cag cct tct tgc ctg ggg acc atg ccc ctg tct      1825
Asp Tyr Arg Arg Leu Gln Pro Ser Cys Leu Gly Thr Met Pro Leu Ser
590                 595                 600                 605

gtg tgc cca ccc atg gct gag tca ggg tcc tgc tgt acc acc cac att      1873
Val Cys Pro Pro Met Ala Glu Ser Gly Ser Cys Cys Thr Thr His Ile
                610                 615                 620

gcc aac cat tcc tac cta cca cta agc tat tgg cag cag cct tga          1918
Ala Asn His Ser Tyr Leu Pro Leu Ser Tyr Trp Gln Gln Pro
            625                 630                 635

gtcgac                                                               1924

<210>  10
<211>  635
<212>  PRT
<213>  Homo sapiens

<400> 10

Met Pro Ser Trp Ala Leu Phe Met Val Thr Ser Cys Leu Leu Leu Ala

1               5                   10                  15

Pro Gln Asn Leu Ala Gln Val Ser Ser Gln Asp Val Ser Leu Leu Ala

            20                  25                  30

Ser Asp Ser Glu Pro Leu Lys Cys Phe Ser Arg Thr Phe Glu Asp Leu

        35                  40                  45

Thr Cys Phe Trp Asp Glu Glu Glu Ala Ala Pro Ser Gly Thr Tyr Gln

    50                  55                  60

Leu Leu Tyr Ala Tyr Pro Arg Glu Lys Pro Arg Ala Cys Pro Leu Ser

65                  70                  75                  80

Ser Gln Ser Met Pro His Phe Gly Thr Arg Tyr Val Cys Gln Phe Pro

                85                  90                  95

Asp Gln Glu Glu Val Arg Leu Phe Phe Pro Leu His Leu Trp Val Lys

            100                 105                 110

Asn Val Phe Leu Asn Gln Thr Arg Thr Gln Arg Val Leu Phe Val Asp

        115                 120                 125

Ser Val Gly Leu Pro Ala Pro Pro Ser Ile Ile Lys Ala Met Gly Gly

    130                 135                 140

Ser Gln Pro Gly Glu Leu Gln Ile Ser Trp Glu Glu Pro Ala Pro Glu

145                 150                 155                 160

Ile Ser Asp Phe Leu Arg Tyr Glu Leu Arg Tyr Gly Pro Arg Asp Pro

                165                 170                 175

Lys Asn Ser Thr Gly Pro Thr Val Ile Gln Leu Ile Ala Thr Glu Thr

            180                 185                 190

Cys Cys Pro Ala Leu Gln Arg Pro His Ser Ala Ser Ala Leu Asp Gln

        195                 200                 205

Ser Pro Cys Ala Gln Pro Thr Met Pro Trp Gln Asp Gly Pro Lys Gln

    210                 215                 220

Thr Ser Pro Ser Arg Glu Ala Ser Ala Leu Thr Ala Glu Gly Gly Ser

225                 230                 235                 240

Cys Leu Ile Ser Gly Leu Gln Pro Gly Asn Ser Tyr Trp Leu Gln Leu

                245                 250                 255

Cys Ser Glu Pro Asp Gly Ile Ser Leu Gly Gly Ser Trp Gly Ser Trp

            260                 265                 270

Ser Leu Pro Val Thr Val Asp Leu Pro Gly Asp Ala Val Ala Leu Gly
        275                 280                 285
Leu Gln Cys Phe Thr Leu Asp Leu Lys Asn Val Thr Cys Gln Trp Gln
    290                 295                 300
Gln Gln Asp His Ala Ser Ser Gln Gly Phe Phe Tyr His Ser Arg Ala
305                 310                 315                 320
Arg Cys Cys Pro Arg Asp Arg Tyr Pro Ile Trp Glu Asn Cys Glu Glu
                325                 330                 335
Glu Glu Lys Thr Asn Pro Gly Leu Gln Thr Pro Gln Phe Ser Arg Cys
            340                 345                 350
His Phe Lys Ser Arg Asn Asp Ser Ile Ile His Ile Leu Val Glu Val
        355                 360                 365
Thr Thr Ala Pro Gly Thr Val His Ser Tyr Leu Gly Ser Pro Phe Trp
    370                 375                 380
Ile His Gln Ala Val Arg Leu Pro Thr Pro Asn Leu His Trp Arg Glu
385                 390                 395                 400
Ile Ser Ser Gly His Leu Glu Leu Glu Trp Gln His Pro Ser Ser Trp
                405                 410                 415

Ala Ala Gln Glu Thr Cys Tyr Gln Leu Arg Tyr Thr Gly Glu Gly His
            420                 425                 430
Gln Asp Trp Lys Val Leu Glu Pro Pro Leu Gly Ala Arg Gly Gly Thr
        435                 440                 445
Leu Glu Leu Arg Pro Arg Ser Arg Tyr Arg Leu Gln Leu Arg Ala Arg
    450                 455                 460
Leu Asn Gly Pro Thr Tyr Gln Gly Pro Trp Ser Ser Trp Ser Asp Pro
465                 470                 475                 480
Thr Arg Val Glu Thr Ala Thr Glu Thr Ala Trp Ile Ser Leu Val Thr
                485                 490                 495
Ala Leu His Leu Val Leu Gly Leu Ser Ala Val Leu Gly Leu Leu Leu
            500                 505                 510
Leu Arg Trp Gln Phe Pro Ala His Tyr Arg Arg Leu Arg His Ala Leu
        515                 520                 525
Trp Pro Ser Leu Pro Asp Leu His Arg Val Leu Gly Gln Tyr Leu Arg
    530                 535                 540
Asp Thr Ala Ala Leu Ser Pro Pro Lys Ala Thr Val Ser Asp Thr Cys

545                 550                 555                 560

Glu Glu Val Glu Pro Ser Leu Leu Glu Ile Leu Pro Lys Ser Ser Glu
                565                 570                 575

Arg Thr Pro Leu Pro Leu Cys Ser Ser Gln Ala Gln Met Asp Tyr Arg
            580                 585                 590

Arg Leu Gln Pro Ser Cys Leu Gly Thr Met Pro Leu Ser Val Cys Pro
        595                 600                 605

Pro Met Ala Glu Ser Gly Ser Cys Cys Thr Thr His Ile Ala Asn His
    610                 615                 620

Ser Tyr Leu Pro Leu Ser Tyr Trp Gln Gln Pro
625                 630                 635

<210>  11
<211>  1924
<212>  DNA
<213>  Homo sapiens

<220>
<221>  CDS
<222>  (11)..(1918)

<223>

<400> 11

gaattccacc atg ccc tcc tgg gcc ctc ttc atg gtc acc tcc tgc ctc 49

Met Pro Ser Trp Ala Leu Phe Met Val Thr Ser Cys Leu

1               5                   10

ctc ctg gcc cct caa aac ctg gcc caa gtc agc agc caa gat gtc tcc 97

Leu Leu Ala Pro Gln Asn Leu Ala Gln Val Ser Ser Gln Asp Val Ser

    15                  20                  25

ttg ctg gca tca gac tca gag ccc ctg aag tgt ttc tcc cga aca ttt 145

Leu Leu Ala Ser Asp Ser Glu Pro Leu Lys Cys Phe Ser Arg Thr Phe

30                  35                  40                  45

gag gac ctc act tgc ttc tgg gat gag gaa gag gca gcg ccc agt ggg 193

Glu Asp Leu Thr Cys Phe Trp Asp Glu Glu Glu Ala Ala Pro Ser Gly

                50                  55                  60

aca tac cag ctg ctg tat gcc tac ccg cgg gag aag ccc cgt gct tgc 241

Thr Tyr Gln Leu Leu Tyr Ala Tyr Pro Arg Glu Lys Pro Arg Ala Cys

            65                  70                  75

ccc ctg agt tcc cag agc atg ccc cac ttt gga acc cga tac gtg tgc 289

Pro Leu Ser Ser Gln Ser Met Pro His Phe Gly Thr Arg Tyr Val Cys

        80                  85                  90

cag ttt cca gac cag gag gaa gtg cgt ctc ttc ttt ccg ctg cac ctc 337
Gln Phe Pro Asp Gln Glu Glu Val Arg Leu Phe Phe Pro Leu His Leu
    95                  100                 105

tgg gtg aag aat gtg ttc cta aac cag act cgg act cag cga gtc ctc 385
Trp Val Lys Asn Val Phe Leu Asn Gln Thr Arg Thr Gln Arg Val Leu
110                 115                 120                 125

ttt gtg gac agt gta ggc ctg ccg gct ccc ccc agt atc atc aag gcc 433
Phe Val Asp Ser Val Gly Leu Pro Ala Pro Pro Ser Ile Ile Lys Ala
                130                 135                 140

atg ggt ggg agc cag cca ggg gaa ctt cag atc agc tgg gag gag cca 481
Met Gly Gly Ser Gln Pro Gly Glu Leu Gln Ile Ser Trp Glu Glu Pro
            145                 150                 155

gct cca gaa atc agt gat ttc ctg agg tac gaa ctc cgc tat ggc ccc 529
Ala Pro Glu Ile Ser Asp Phe Leu Arg Tyr Glu Leu Arg Tyr Gly Pro
        160                 165                 170

aga gat ccc aag aac tcc act ggt ccc acg gtc ata cag ctg att gcc 577
Arg Asp Pro Lys Asn Ser Thr Gly Pro Thr Val Ile Gln Leu Ile Ala
    175                 180                 185

aca gaa acc tgc tgc cct gct ctg cag aga cct cac tca gcc tct gct 625

Thr Glu Thr Cys Cys Pro Ala Leu Gln Arg Pro His Ser Ala Ser Ala
190                 195                 200                 205

ctg gac cag tct cca tgt gct cag ccc aca atg ccc tgg caa gat gga 673
Leu Asp Gln Ser Pro Cys Ala Gln Pro Thr Met Pro Trp Gln Asp Gly
                210                 215                 220

cca aag cag acc tcc cca agt aga gaa gct tca gct ctg aca gca gag 721
Pro Lys Gln Thr Ser Pro Ser Arg Glu Ala Ser Ala Leu Thr Ala Glu
            225                 230                 235

ggt gga agc tgc ctc atc tca gga ctc cag cct ggc aac tcc tac tgg 769
Gly Gly Ser Cys Leu Ile Ser Gly Leu Gln Pro Gly Asn Ser Tyr Trp
        240                 245                 250

ctg cag ctg cgc agc gaa cct gat ggg atc tcc ctc ggt ggc tcc tgg 817
Leu Gln Leu Arg Ser Glu Pro Asp Gly Ile Ser Leu Gly Gly Ser Trp
    255                 260                 265

gga tcc tgg tcc ctc act gtg act gtg gac ctg cct gga gat gca gtg 865
Gly Ser Trp Ser Leu Thr Val Thr Val Asp Leu Pro Gly Asp Ala Val
270                 275                 280                 285

gca ctt gga ctg caa tgc ttt acc ttg gac ctg aag aat gtt acc tgt 913
Ala Leu Gly Leu Gln Cys Phe Thr Leu Asp Leu Lys Asn Val Thr Cys
                290                 295                 300

caa tgg cag caa cag gac cat gct agc tcc caa ggc ttc ttc tac cac 961
Gln Trp Gln Gln Gln Asp His Ala Ser Ser Gln Gly Phe Phe Tyr His
            305                 310                 315

agc agg gca cgg tgc tgc ccc aga gac agg tac ccc atc tgg gag aac 1009
Ser Arg Ala Arg Cys Cys Pro Arg Asp Arg Tyr Pro Ile Trp Glu Asn
        320                 325                 330

tgc gaa gag gaa gag aaa aca aat cca gga cta cag acc cca cag ttc 1057
Cys Glu Glu Glu Glu Lys Thr Asn Pro Gly Leu Gln Thr Pro Gln Phe
    335                 340                 345

tct cgc tgc cac ttc aag tca cga aat gac agc att att cac atc ctt 1105
Ser Arg Cys His Phe Lys Ser Arg Asn Asp Ser Ile Ile His Ile Leu
350                 355                 360                 365

gtg gag gtg acc aca gcc ccg ggt act gtt cac agc tac ctg ggc tcc 1153
Val Glu Val Thr Thr Ala Pro Gly Thr Val His Ser Tyr Leu Gly Ser
                370                 375                 380

cct ttc tgg atc cac cag gct gtg cgc ctc ccc acc cca aac ttg cac 1201
Pro Phe Trp Ile His Gln Ala Val Arg Leu Pro Thr Pro Asn Leu His
            385                 390                 395

tgg agg gag atc tcc agt ggg cat ctg gaa ttg gag tgg cag cac cca 1249

Trp Arg Glu Ile Ser Ser Gly His Leu Glu Leu Glu Trp Gln His Pro
        400                 405                 410

tcg tcc tgg gca gcc caa gag acc tgt tat caa ctc cga tac aca gga 1297
Ser Ser Trp Ala Ala Gln Glu Thr Cys Tyr Gln Leu Arg Tyr Thr Gly
    415                 420                 425

gaa ggc cat cag gac tgg aag gtg ctg gag ccg cct ctc ggg gcc cga 1345
Glu Gly His Gln Asp Trp Lys Val Leu Glu Pro Pro Leu Gly Ala Arg
430                 435                 440                 445

gga ggg acc ctg gag ctg cgc ccg cga tct cgc tac cgt tta cag ctg 1393
Gly Gly Thr Leu Glu Leu Arg Pro Arg Ser Arg Tyr Arg Leu Gln Leu
                450                 455                 460

cgc gcc agg ctc aac ggc ccc acc tac caa ggt ccc tgg agc tcg tgg 1441
Arg Ala Arg Leu Asn Gly Pro Thr Tyr Gln Gly Pro Trp Ser Ser Trp
            465                 470                 475

tcg gac cca act agg gtg gag acc gcc acc gag acc gcc tgg atc tcc 1489
Ser Asp Pro Thr Arg Val Glu Thr Ala Thr Glu Thr Ala Trp Ile Ser
        480                 485                 490

ttg gtg acc gct ctg cat cta gtg ctg ggc ctc agc gcc gtc ctg ggc 1537
Leu Val Thr Ala Leu His Leu Val Leu Gly Leu Ser Ala Val Leu Gly
    495                 500                 505

ctg ctg ctg ctg agg tgg cag ttt cct gca cac tac agg aga ctg agg 1585
Leu Leu Leu Leu Arg Trp Gln Phe Pro Ala His Tyr Arg Arg Leu Arg
510                 515                 520                 525

cat gcc ctg tgg ccc tca ctt cca gac ctg cac cgg gtc cta ggc cag 1633
His Ala Leu Trp Pro Ser Leu Pro Asp Leu His Arg Val Leu Gly Gln
                530                 535                 540

tac ctt agg gac act gca gcc ctg agc ccg ccc aag gcc aca gtc tca 1681
Tyr Leu Arg Asp Thr Ala Ala Leu Ser Pro Pro Lys Ala Thr Val Ser
            545                 550                 555

gat acc tgt gaa gaa gtg gaa ccc agc ctc ctt gaa atc ctc ccc aag 1729
Asp Thr Cys Glu Glu Val Glu Pro Ser Leu Leu Glu Ile Leu Pro Lys
        560                 565                 570

tcc tca gag agg act cct ttg ccc ctg tgt tcc tcc cag gcc cag atg 1777
Ser Ser Glu Arg Thr Pro Leu Pro Leu Cys Ser Ser Gln Ala Gln Met
    575                 580                 585

gac tac cga aga ttg cag cct tct tgc ctg ggg acc atg ccc ctg tct 1825
Asp Tyr Arg Arg Leu Gln Pro Ser Cys Leu Gly Thr Met Pro Leu Ser
590                 595                 600                 605

gtg tgc cca ccc atg gct gag tca ggg tcc tgc tgt acc acc cac att 1873

Val Cys Pro Pro Met Ala Glu Ser Gly Ser Cys Cys Thr Thr His Ile
                610                 615                 620

gcc aac cat tcc tac cta cca cta agc tat tgg cag cag cct tga 1918
Ala Asn His Ser Tyr Leu Pro Leu Ser Tyr Trp Gln Gln Pro
            625                 630                 635

gtcgac 1924

<210> 12
<211> 635
<212> PRT
<213> Homo sapiens

<400> 12
Met Pro Ser Trp Ala Leu Phe Met Val Thr Ser Cys Leu Leu Leu Ala
1               5                   10                  15

Pro Gln Asn Leu Ala Gln Val Ser Ser Gln Asp Val Ser Leu Leu Ala
            20                  25                  30

Ser Asp Ser Glu Pro Leu Lys Cys Phe Ser Arg Thr Phe Glu Asp Leu
        35                  40                  45

Thr Cys Phe Trp Asp Glu Glu Glu Ala Ala Pro Ser Gly Thr Tyr Gln

    50                  55                  60

Leu Leu Tyr Ala Tyr Pro Arg Glu Lys Pro Arg Ala Cys Pro Leu Ser
65                  70                  75                  80

Ser Gln Ser Met Pro His Phe Gly Thr Arg Tyr Val Cys Gln Phe Pro
                85                  90                  95

Asp Gln Glu Glu Val Arg Leu Phe Phe Pro Leu His Leu Trp Val Lys
            100                 105                 110

Asn Val Phe Leu Asn Gln Thr Arg Thr Gln Arg Val Leu Phe Val Asp
        115                 120                 125

Ser Val Gly Leu Pro Ala Pro Pro Ser Ile Ile Lys Ala Met Gly Gly
    130                 135                 140

Ser Gln Pro Gly Glu Leu Gln Ile Ser Trp Glu Glu Pro Ala Pro Glu
145                 150                 155                 160

Ile Ser Asp Phe Leu Arg Tyr Glu Leu Arg Tyr Gly Pro Arg Asp Pro
                165                 170                 175

Lys Asn Ser Thr Gly Pro Thr Val Ile Gln Leu Ile Ala Thr Glu Thr
            180                 185                 190

Cys Cys Pro Ala Leu Gln Arg Pro His Ser Ala Ser Ala Leu Asp Gln
        195                 200                 205
Ser Pro Cys Ala Gln Pro Thr Met Pro Trp Gln Asp Gly Pro Lys Gln
    210                 215                 220
Thr Ser Pro Ser Arg Glu Ala Ser Ala Leu Thr Ala Glu Gly Gly Ser
225                 230                 235                 240
Cys Leu Ile Ser Gly Leu Gln Pro Gly Asn Ser Tyr Trp Leu Gln Leu
                245                 250                 255
Arg Ser Glu Pro Asp Gly Ile Ser Leu Gly Gly Ser Trp Gly Ser Trp
            260                 265                 270
Ser Leu Thr Val Thr Val Asp Leu Pro Gly Asp Ala Val Ala Leu Gly
        275                 280                 285
Leu Gln Cys Phe Thr Leu Asp Leu Lys Asn Val Thr Cys Gln Trp Gln
    290                 295                 300
Gln Gln Asp His Ala Ser Ser Gln Gly Phe Phe Tyr His Ser Arg Ala
305                 310                 315                 320
Arg Cys Cys Pro Arg Asp Arg Tyr Pro Ile Trp Glu Asn Cys Glu Glu
                325                 330                 335

Glu Glu Lys Thr Asn Pro Gly Leu Gln Thr Pro Gln Phe Ser Arg Cys
            340                 345                 350
His Phe Lys Ser Arg Asn Asp Ser Ile Ile His Ile Leu Val Glu Val
        355                 360                 365
Thr Thr Ala Pro Gly Thr Val His Ser Tyr Leu Gly Ser Pro Phe Trp
    370                 375                 380
Ile His Gln Ala Val Arg Leu Pro Thr Pro Asn Leu His Trp Arg Glu
385                 390                 395                 400
Ile Ser Ser Gly His Leu Glu Leu Glu Trp Gln His Pro Ser Ser Trp
                405                 410                 415
Ala Ala Gln Glu Thr Cys Tyr Gln Leu Arg Tyr Thr Gly Glu Gly His
            420                 425                 430
Gln Asp Trp Lys Val Leu Glu Pro Pro Leu Gly Ala Arg Gly Gly Thr
        435                 440                 445
Leu Glu Leu Arg Pro Arg Ser Arg Tyr Arg Leu Gln Leu Arg Ala Arg
    450                 455                 460
Leu Asn Gly Pro Thr Tyr Gln Gly Pro Trp Ser Ser Trp Ser Asp Pro

465                 470                 475                 480

Thr Arg Val Glu Thr Ala Thr Glu Thr Ala Trp Ile Ser Leu Val Thr

                485                 490                 495

Ala Leu His Leu Val Leu Gly Leu Ser Ala Val Leu Gly Leu Leu Leu

            500                 505                 510

Leu Arg Trp Gln Phe Pro Ala His Tyr Arg Arg Leu Arg His Ala Leu

        515                 520                 525

Trp Pro Ser Leu Pro Asp Leu His Arg Val Leu Gly Gln Tyr Leu Arg

    530                 535                 540

Asp Thr Ala Ala Leu Ser Pro Pro Lys Ala Thr Val Ser Asp Thr Cys

545                 550                 555                 560

Glu Glu Val Glu Pro Ser Leu Leu Glu Ile Leu Pro Lys Ser Ser Glu

                565                 570                 575

Arg Thr Pro Leu Pro Leu Cys Ser Ser Gln Ala Gln Met Asp Tyr Arg

            580                 585                 590

Arg Leu Gln Pro Ser Cys Leu Gly Thr Met Pro Leu Ser Val Cys Pro

        595                 600                 605

Pro Met Ala Glu Ser Gly Ser Cys Cys Thr Thr His Ile Ala Asn His
610                 615                 620

Ser Tyr Leu Pro Leu Ser Tyr Trp Gln Gln Pro
625                 630                 635

<210>  13
<211>  1560
<212>  DNA
<213>  Mus musculus

<220>
<221>  CDS
<222>  (1)..(1560)
<223>

<400>  13
atg aga gtg ctg att cct ttg tgg ctg ttc aca gcc ttt cct ggt acc        48
Met Arg Val Leu Ile Pro Leu Trp Leu Phe Thr Ala Phe Pro Gly Thr
1               5                   10                  15

ctg tct gat gtg cag ctg cag gag tcg gga cct ggc ctg gtg aaa cct        96
Leu Ser Asp Val Gln Leu Gln Glu Ser Gly Pro Gly Leu Val Lys Pro
            20                  25                  30

tct cag tct ctg tcc ctc acc tgc act gtc act ggc tac tca atc acc 144
Ser Gln Ser Leu Ser Leu Thr Cys Thr Val Thr Gly Tyr Ser Ile Thr
        35                  40                  45

agt gat tat gcc tgg agc tgg atc cga cag tta cca gga aac aaa ctg 192
Ser Asp Tyr Ala Trp Ser Trp Ile Arg Gln Leu Pro Gly Asn Lys Leu
    50                  55                  60

gag tgg atg ggc tac ata acg tac agt ggt tac tct atc tac aat cca 240
Glu Trp Met Gly Tyr Ile Thr Tyr Ser Gly Tyr Ser Ile Tyr Asn Pro
65                  70                  75                  80

tct ctc aaa agt cga atc tct atc agt cga gac aca tcc aag aac cag 288
Ser Leu Lys Ser Arg Ile Ser Ile Ser Arg Asp Thr Ser Lys Asn Gln
                85                  90                  95

ttg ttc ctg cag tta aat tct gtg acc act gag gac aca gcc aca tat 336
Leu Phe Leu Gln Leu Asn Ser Val Thr Thr Glu Asp Thr Ala Thr Tyr
            100                 105                 110

tac tgt gtg ggc ggg tat gac aat atg gac tat tgg ggt caa gga acc 384
Tyr Cys Val Gly Gly Tyr Asp Asn Met Asp Tyr Trp Gly Gln Gly Thr
        115                 120                 125

tca gtc acc gtc tcc tca ggt ggt ggt ggt tcg ggt ggt ggt ggt tcg 432
Ser Val Thr Val Ser Ser Gly Gly Gly Gly Ser Gly Gly Gly Gly Ser

130                 135                 140

ggt ggt ggc gga tcg caa att gtt ctc acc cag tct cca gca atc atg        480
Gly Gly Gly Gly Ser Gln Ile Val Leu Thr Gln Ser Pro Ala Ile Met
145                 150                 155                 160

tct gca tct cct ggg gag aag gtc acc ttg acc tgc agt gcc agc tca        528
Ser Ala Ser Pro Gly Glu Lys Val Thr Leu Thr Cys Ser Ala Ser Ser
                165                 170                 175

agt gta agt tcc agc cac tta tac tgg tat cag cag aag cca ggg tcc        576
Ser Val Ser Ser Ser His Leu Tyr Trp Tyr Gln Gln Lys Pro Gly Ser
            180                 185                 190

tcc ccc aaa ctc tgg att tat agc aca tcc aac ctg gct tct gga gtc        624
Ser Pro Lys Leu Trp Ile Tyr Ser Thr Ser Asn Leu Ala Ser Gly Val
        195                 200                 205

cct gct cgc ttc agt ggc agt ggg tct ggg acc tcc tat tct ctc aca        672
Pro Ala Arg Phe Ser Gly Ser Gly Ser Gly Thr Ser Tyr Ser Leu Thr
    210                 215                 220

atc agc aac atg gag act gaa gat gct gcc tct tat ttc tgc cat cag        720
Ile Ser Asn Met Glu Thr Glu Asp Ala Ala Ser Tyr Phe Cys His Gln
225                 230                 235                 240

tgg agt agt tac cca tgg acg ttc ggt ggg ggc acc aag ctg gaa atc 768
Trp Ser Ser Tyr Pro Trp Thr Phe Gly Gly Gly Thr Lys Leu Glu Ile
                245                 250                 255

aaa gga ggt ggt gga tcg ggt ggt ggt ggt tcg gga ggc ggt gga tcg 816
Lys Gly Gly Gly Gly Ser Gly Gly Gly Gly Ser Gly Gly Gly Gly Ser
            260                 265                 270

gat gtg cag ctg cag gag tcg gga cct ggc ctg gtg aaa cct tct cag 864
Asp Val Gln Leu Gln Glu Ser Gly Pro Gly Leu Val Lys Pro Ser Gln
        275                 280                 285

tct ctg tcc ctc acc tgc act gtc act ggc tac tca atc acc agt gat 912
Ser Leu Ser Leu Thr Cys Thr Val Thr Gly Tyr Ser Ile Thr Ser Asp
    290                 295                 300

tat gcc tgg agc tgg atc cga cag tta cca gga aac aaa ctg gag tgg 960
Tyr Ala Trp Ser Trp Ile Arg Gln Leu Pro Gly Asn Lys Leu Glu Trp
305                 310                 315                 320

atg ggc tac ata acg tac agt ggt tac tct atc tac aat cca tct ctc 1008
Met Gly Tyr Ile Thr Tyr Ser Gly Tyr Ser Ile Tyr Asn Pro Ser Leu
                325                 330                 335

aaa agt cga atc tct atc agt cga gac aca tcc aag aac cag ttg ttc 1056
Lys Ser Arg Ile Ser Ile Ser Arg Asp Thr Ser Lys Asn Gln Leu Phe

            340                 345                 350

ctg cag tta aat tct gtg acc act gag gac aca gcc aca tat tac tgt 1104
Leu Gln Leu Asn Ser Val Thr Thr Glu Asp Thr Ala Thr Tyr Tyr Cys
        355                 360                 365

gtg ggc ggg tat gac aat atg gac tat tgg ggt caa gga acc tca gtc 1152
Val Gly Gly Tyr Asp Asn Met Asp Tyr Trp Gly Gln Gly Thr Ser Val
    370                 375                 380

acc gtc tcc tca ggt ggt ggt ggt tcg ggt ggt ggt ggt tcg ggt ggt 1200
Thr Val Ser Ser Gly Gly Gly Gly Ser Gly Gly Gly Gly Ser Gly Gly
385                 390                 395                 400

ggc gga tcg caa att gtt ctc acc cag tct cca gca atc atg tct gca 1248
Gly Gly Ser Gln Ile Val Leu Thr Gln Ser Pro Ala Ile Met Ser Ala
                405                 410                 415

tct cct ggg gag aag gtc acc ttg acc tgc agt gcc agc tca agt gta 1296
Ser Pro Gly Glu Lys Val Thr Leu Thr Cys Ser Ala Ser Ser Ser Val
            420                 425                 430

agt tcc agc cac tta tac tgg tat cag cag aag cca ggg tcc tcc ccc 1344
Ser Ser Ser His Leu Tyr Trp Tyr Gln Gln Lys Pro Gly Ser Ser Pro
        435                 440                 445

aaa ctc tgg att tat agc aca tcc aac ctg gct tct gga gtc cct gct 1392
Lys Leu Trp Ile Tyr Ser Thr Ser Asn Leu Ala Ser Gly Val Pro Ala
    450                 455                 460

cgc ttc agt ggc agt ggg tct ggg acc tcc tat tct ctc aca atc agc 1440
Arg Phe Ser Gly Ser Gly Ser Gly Thr Ser Tyr Ser Leu Thr Ile Ser
465                 470                 475                 480

aac atg gag act gaa gat gct gcc tct tat ttc tgc cat cag tgg agt 1488
Asn Met Glu Thr Glu Asp Ala Ala Ser Tyr Phe Cys His Gln Trp Ser
                485                 490                 495

agt tac cca tgg acg ttc ggt ggg ggc acc aag ctg gaa atc aaa gac 1536
Ser Tyr Pro Trp Thr Phe Gly Gly Gly Thr Lys Leu Glu Ile Lys Asp
            500                 505                 510

tac aag gat gac gac gat aag tga 1560
Tyr Lys Asp Asp Asp Asp Lys
        515

<210> 14
<211> 519
<212> PRT
<213> Mus musculus

<400> 14

Met Arg Val Leu Ile Pro Leu Trp Leu Phe Thr Ala Phe Pro Gly Thr

1               5                   10                  15

Leu Ser Asp Val Gln Leu Gln Glu Ser Gly Pro Gly Leu Val Lys Pro

            20                  25                  30

Ser Gln Ser Leu Ser Leu Thr Cys Thr Val Thr Gly Tyr Ser Ile Thr

        35                  40                  45

Ser Asp Tyr Ala Trp Ser Trp Ile Arg Gln Leu Pro Gly Asn Lys Leu

    50                  55                  60

Glu Trp Met Gly Tyr Ile Thr Tyr Ser Gly Tyr Ser Ile Tyr Asn Pro

65                  70                  75                  80

Ser Leu Lys Ser Arg Ile Ser Ile Ser Arg Asp Thr Ser Lys Asn Gln

                85                  90                  95

Leu Phe Leu Gln Leu Asn Ser Val Thr Thr Glu Asp Thr Ala Thr Tyr

            100                 105                 110

Tyr Cys Val Gly Gly Tyr Asp Asn Met Asp Tyr Trp Gly Gln Gly Thr

        115                 120                 125

Ser Val Thr Val Ser Ser Gly Gly Gly Gly Ser Gly Gly Gly Gly Ser

    130                 135                 140

Gly Gly Gly Gly Ser Gln Ile Val Leu Thr Gln Ser Pro Ala Ile Met
145                 150                 155                 160

Ser Ala Ser Pro Gly Glu Lys Val Thr Leu Thr Cys Ser Ala Ser Ser
                165                 170                 175

Ser Val Ser Ser Ser His Leu Tyr Trp Tyr Gln Gln Lys Pro Gly Ser
            180                 185                 190

Ser Pro Lys Leu Trp Ile Tyr Ser Thr Ser Asn Leu Ala Ser Gly Val
        195                 200                 205

Pro Ala Arg Phe Ser Gly Ser Gly Ser Gly Thr Ser Tyr Ser Leu Thr
    210                 215                 220

Ile Ser Asn Met Glu Thr Glu Asp Ala Ala Ser Tyr Phe Cys His Gln
225                 230                 235                 240

Trp Ser Ser Tyr Pro Trp Thr Phe Gly Gly Gly Thr Lys Leu Glu Ile
                245                 250                 255

Lys Gly Gly Gly Gly Ser Gly Gly Gly Gly Ser Gly Gly Gly Gly Ser
            260                 265                 270

Asp Val Gln Leu Gln Glu Ser Gly Pro Gly Leu Val Lys Pro Ser Gln
        275                 280                 285
Ser Leu Ser Leu Thr Cys Thr Val Thr Gly Tyr Ser Ile Thr Ser Asp
    290                 295                 300
Tyr Ala Trp Ser Trp Ile Arg Gln Leu Pro Gly Asn Lys Leu Glu Trp
305                 310                 315                 320
Met Gly Tyr Ile Thr Tyr Ser Gly Tyr Ser Ile Tyr Asn Pro Ser Leu
                325                 330                 335
Lys Ser Arg Ile Ser Ile Ser Arg Asp Thr Ser Lys Asn Gln Leu Phe
            340                 345                 350
Leu Gln Leu Asn Ser Val Thr Thr Glu Asp Thr Ala Thr Tyr Tyr Cys
        355                 360                 365
Val Gly Gly Tyr Asp Asn Met Asp Tyr Trp Gly Gln Gly Thr Ser Val
    370                 375                 380
Thr Val Ser Ser Gly Gly Gly Gly Ser Gly Gly Gly Gly Ser Gly Gly
385                 390                 395                 400
Gly Gly Ser Gln Ile Val Leu Thr Gln Ser Pro Ala Ile Met Ser Ala
                405                 410                 415

Ser Pro Gly Glu Lys Val Thr Leu Thr Cys Ser Ala Ser Ser Ser Val
            420                 425                 430
Ser Ser Ser His Leu Tyr Trp Tyr Gln Gln Lys Pro Gly Ser Ser Pro
        435                 440                 445
Lys Leu Trp Ile Tyr Ser Thr Ser Asn Leu Ala Ser Gly Val Pro Ala
    450                 455                 460
Arg Phe Ser Gly Ser Gly Ser Gly Thr Ser Tyr Ser Leu Thr Ile Ser
465                 470                 475                 480
Asn Met Glu Thr Glu Asp Ala Ala Ser Tyr Phe Cys His Gln Trp Ser
                485                 490                 495
Ser Tyr Pro Trp Thr Phe Gly Gly Gly Thr Lys Leu Glu Ile Lys Asp
            500                 505                 510
Tyr Lys Asp Asp Asp Asp Lys
        515
<210> 15
<211> 82
<212> DNA

<213>  Artificial

<220>

<223>  an artificially synthesized primer sequence

<400>  15

tagaattcca ccatgagagt gctgattcct ttgtggctgt tcacagcctt tcctggtacc      60

ctgtctgatg tgcagctgca gg                                                82

<210>  16

<211>  82

<212>  DNA

<213>  Artificial

<220>

<223>  an artificially synthesized primer sequence

<400>  16

tgggtgagaa caatttgcga tccgccacca cccgaaccac caccacccga accaccacca      60

cctgaggaga cggtgactga gg                                                82

<210>  17

<211> 82

<212> DNA

<213> Artificial

<220>

<223> an artificially synthesized primer sequence

<400> 17

cagtcaccgt ctcctcaggt ggtggtggtt cgggtggtgg tggttcgggt ggtggcggat 60

cgcaaattgt tctcacccag tc 82

<210> 18

<211> 60

<212> DNA

<213> Artificial

<220>

<223> an artificially synthesized primer sequence

<400> 18

attgcggccg cttatcactt atcgtcgtca tccttgtagt ctttgatttc cagcttggtg 60

<210> 19

<211>  25

<212>  DNA

<213>  Artificial

<220>

<223>  an artificially synthesized primer sequence

<400>  19
gatgtgcagc tgcaggagtc gggac                                          25

<210>  20

<211>  81

<212>  DNA

<213>  Artificial

<220>

<223>  an artificially synthesized primer sequence

<400>  20
cctgcagctg cacatccgat ccaccgcctc ccgaaccacc accacccgat ccaccacctc    60

ctttgatttc cagcttggtg c                                              81

## Description

### Technical Field

The present invention relates to ligands having agonistic activity to mutant receptors, and pharmaceutical compositions comprising the ligands as active ingredients.

### Background Art

In recent years, the causative genes of various diseases have been identified in quick succession, and a variety of therapeutic methods for such diseases have been studied and established. Of these methods, the most intensively studied are mostly therapeutic methods for complementing enzyme gene deficiencies. It has been reported that enzyme replacement therapy using "Cerezyme" (Genzyme) is effective for patients with Gaucher's disease, in which β-glucocerebrosidase is deficient, and that enzyme replacement therapy using "Aldurazyme" (Genzyme) is effective for patients with mucopolysaccharidosis, in which α-L-iduronidase is deficient. Previously attempted gene therapies include introducing the adenosine deaminase (ADA) gene to patients with ADA deficiency, and introducing the coagulation factor IX gene to patients with hemophilia B. In addition to enzyme deficiencies, a large number of genetic diseases are known, such as genetic diseases of cytokines and their receptors. Some patients with type II diabetes mellitus, which accounts for approximately 90% of diabetes mellitus cases, have been reported to have insulin receptor deletions or mutations. Such deletions and mutations are assumed to cause the disease. Furthermore, some patients with thrombocytopenia have been reported to have thrombopoietin receptor deletions and mutations, and the failure of TPO signaling can be thought to cause the disease. To date, no fundamental therapeutic methods have been available for such genetic diseases, and the establishment of such therapeutic methods is expected.

Congenital amegakaryocytic thrombocytopenia (CAMT) is a rare disorder that causes thrombocytopenia in infancy and pancytopenia in later childhood. It has been revealed that TPO, a thrombopoietic growth factor, is present in CAMT patients at a high concentration in sera, but that platelets and hematopoietic precursor cells lack TPO responsiveness. Most of these patients have been found to carry point mutations in their thrombopoietin receptor (c-MPL) gene. It has also been reported that such mutations result in frame shift or insertion of a termination codon, leading to patients who have a total loss of thrombopoietin receptor function and patients who have a homozygous or heterozygous amino acid substitution in the extracellular domain of the receptor (see Non-patent Document 1). Bone marrow transplant is the only currently available therapeutic method for treating such patients.

### [Non-patent Document 1]

Matthias Ballmaier, Manuela Germeshausen, Harald Schulze, Klara Cherkaoui, Sabine Lang, Annika Gaudig, Stephanie Krukemeier, Martin Eilers, Gabriele Strausz, and Karl Welte, "BLOOD", vol. 97, No. 1, pp. 139 (January 1, 2001).

### Disclosure of the Invention

The present invention was achieved in view of the circumstances described above. An objective of the present invention is to provide ligands having agonistic activity to mutant receptors.

The present inventors studied intensively to achieve the objective described above. By using antibody engineering techniques, the inventors prepared functional antibodies corresponding to each of the mutations in the causative genes of the diseases described above, discovering that these antibodies were useful to treat such diseases. Specifically, the inventors succeeded in preparing minibodies, each of which has agonistic activity to a mutant receptor that has almost completely lost responsiveness to its natural ligand due to gene mutation (for example, a thrombopoietin (TPO) receptor whose responsiveness to TPO is mostly lost), and which can transduce signals at levels comparable to normal levels when reacted with such a mutant receptor.

Diseases caused by gene mutations can be treated by using such antibodies or modified products thereof. Alternatively, such diseases can be treated by gene therapy using genes capable of expressing the antibodies or modified products thereof.

CAMT patients, who were previously difficult to treat, can now be treated using ligands having agonistic activity, such as the antibodies of the present invention or the genes encoding them.

In addition, the methods of the present invention are applicable to various other diseases caused by mutations in genes encoding receptors on cell membranes. Thus, the present invention can provide new therapeutic methods for these diseases.

Specifically, the present invention relates to ligands having agonistic activity to mutant receptors, more specifically provides:
[1] a ligand having agonistic activity to a mutant receptor;
[2] the ligand of [1], where the ligand is an antibody;
[3] the ligand of [1] or [2], where the ligand has greater agonistic activity to the mutant receptor than the natural ligand;
[4]. the ligand of any one of [1] to [3], where the mutant receptor is a receptor resulting from a mutation(s) in the amino acid sequence;
[5] the ligand of any one of [1] to [4], where the mutant receptor has lost responsiveness to the natural ligand;
[6] the ligand of any one of [1] to [5], where the mutant receptor causes a disease;
[7] the ligand of any one of [1] to [6], where the mutant receptor is a mutant thrombopoietin receptor;
[8] the ligand of [2], where the antibody is a minibody;
[9] the ligand of [8], where the minibody is a diabody;
[10] a method for transducing a signal to a mutant receptor by binding a ligand;
[11] the method of [10], where the ligand is an antibody;
[12] the method of [10] or [11], where the mutant receptor results from an amino acid mutation(s);
[13] the method of any one of [10] to [12], where the mutant receptor has lost responsiveness to the natural ligand;
[14] the method of any one of [10] to [13], where the mutant receptor is associated with disease onset;
[15] the method of any one of [10] to [14], where the mutant receptor is a mutant thrombopoietin receptor;
[16] a method for treating a disease caused by a mutant receptor, by binding a ligand to the mutant receptor;
[17] the method of [16], where the ligand is an antibody;
[18] a method of screening for a ligand having agonistic activity to a mutant receptor, where the method comprises the steps of,
   (a) contacting a test substance with the mutant receptor,
   (b) detecting a signal in the mutant receptor, and
   (c) selecting a ligand having agonistic activity;
[19] a method of screening for a ligand having agonistic activity to a mutant receptor, where the method comprises the steps of,
   (a) determining agonistic activity to a normal receptor,
   (b) determining agonistic activity to the mutant receptor, and
   (c) selecting a ligand having greater agonistic activity to the mutant receptor than the normal receptor;
[20] a method of screening for a ligand having agonistic activity to normal and mutant receptors, where the method comprises the steps of,
   (a) determining agonistic activity to the normal receptor,
   (b) determining agonistic activity to the mutant receptor, and
   (c) selecting a ligand having agonistic activity to both normal and mutant receptors;
[21] the method of any one of [18] to [20], where the ligand is an antibody;
[22] a substance obtained by the method of any one of [18] to [21];
[23] a therapeutic agent for a disease caused by a mutant receptor, where the agent comprises a ligand of the mutant receptor; ,
[24] the therapeutic agent of [23], where the ligand is the ligand of any one of [1] to [9];
[25] the therapeutic agent of [23], where the ligand is an antibody;
[26] the therapeutic agent of any one of [23] to [25], where the mutant receptor results from an amino acid mutation(s);
[27] the therapeutic agent of any one of [23] to [26], where the mutant receptor has lost responsiveness to the natural ligand;
[28] the therapeutic agent of any one of [23] to [27], where the mutant receptor is a mutant thrombopoietin receptor; and,
[29] the therapeutic agent of any one of [23] to [28], where the disease is congenital amegakaryocytic thrombocytopenia.

The present invention provides ligands having agonistic activity to mutant receptors.

Mutant receptors of the present invention are usually receptors that exist at a frequency of less than 50%, preferably less than 20%, more preferably less than 10%, and even more preferably less than 1%. The frequency is generally calculated using randomly selected subjects. However, the frequency may vary depending on country, area, sex, and such. Therefore, the frequency may also be calculated, for example, within a selected country or area such as Japan, the United States, or Europe, or be calculated for one sex. When there are two or more mutation sites in a receptor, the frequency may be calculated for multiple mutation sites or for any one of the mutation sites. Mutant receptors are preferably evaluated using frequency, as described above. However, mutant receptors can also be evaluated, for example, by their signal transducing ability. Specifically, for example, when two different receptors are present, the one with stronger transducing signals upon natural ligand-binding may be used as a non-mutant receptor, and the other with weaker transducing signals may be used as a mutant receptor.

Preferred mutant receptors of the present invention include, but are not limited to, receptors resulting from amino acid mutations (receptors with mutated amino acid sequences); however, any type of mutation is acceptable, as long as the mutated receptor influences responsiveness to natural ligands, or the conformation, sugar chain structure, or spatial relationship or angles when a receptor exists as a multimer, and so on. Mutations in the amino acid sequence include amino acid substitutions, deletions, insertions, and additions. The receptors of the present invention have preferably lost responsiveness to the natural ligands.

Herein, "ligand" refers to a substance that specifically binds to a functional protein. The type of ligand is not limited. Such ligands include low-molecular-weight compounds, proteins, and peptides. In the present invention, functional proteins are preferably receptors. In the present invention, ligands preferably have agonistic activity. The present invention also provides methods for transducing signals to a mutant receptor by binding a ligand of the present invention. Such ligands for use in the methods of the present invention are preferably non-natural ligands, and not natural ligands.

In the present invention, it is preferred to target a mutant receptor whose responsiveness to a natural ligand is different from that of a non-mutant receptor. A "mutant receptor whose responsiveness to a natural ligand is different from that of a non-mutant receptor" refers to a mutant receptor that exhibits agonistic activity and signaling activity that differs from the activities of the non-mutant receptor when the mutant and non-mutant receptors bind to the same natural ligand under the same conditions. In general, agonistic activity and signaling activity in mutant receptors is impaired compared with non-mutant receptors (the mutants have lost their responsiveness to natural ligands).

The receptors include receptors belonging to the hematopoietic growth factor receptor family, the cytokine receptor family, the tyrosine kinase receptor family, the serine/threonine kinase receptor family, the TNF receptor family, the G protein-coupled receptor family, the GPI-anchored receptor family, the tyrosine phosphatase receptor family, the adhesion factor family, and the hormone receptor family. There are many documents that describe receptors belonging to these receptor families, and their characteristics; for example Cooke BA, King RJB, van der Molen HJ Eds. New Comprehensive Biochemistry Vol.18B "Hormones and their Actions Part II" pp. 1-46 (1988) Elsevier Science Publishers BV, New York, USA; Patthy L. (1990) Cell, 61: 13-14; Ullrich A. *et al*. (1990) Cell, 61: 203-212; Massagul J. (1992) Cell, 69: 1067-1070; Miyajima A. *et al*. (1992) Annu. Rev. Immunol., 10: 295-331; Taga T. and Kishimoto T. (1992) FASEB J., 7: 3387-3396; Fantl WI. *et al*. (1993) Annu. Rev. Biochem., 62: 453-481; Smith CA., *et al*. (1994) Cell, 76: 959-962; Flower DR. (1999) Biochim. Biophys. Acta, 1422: 207-234; Cell Technology: supplementary vol. Handbook series "Handbook for Adhesion factors" M. Miyasaka Ed. (1994) Shujunnsha, Tokyo, Japan, and so on. Specific receptors belonging to the families listed above include: human and mouse erythropoietin (EPO) receptors, human and mouse granulocyte-colony stimulating factor (G-CSF) receptors, human and mouse thrombopoietin (TPO) receptors, human and mouse insulin receptors, human and mouse Flt-3 ligand receptors, human and mouse platelet-derived growth factor (PDGF) receptors, human and mouse interferon (IFN)-α and -β receptors, human and mouse leptin receptors, human and mouse growth hormone (GH) receptors, human and mouse interleukin (IL)-10 receptors, human and mouse insulin-like growth factor (IGF)-I receptors, human and mouse leukemia inhibitory factor (LIF) receptors, and human and mouse ciliary neurotrophic factor (CNTF) receptors (hEPOR: Simon, S. *et al*. (1990) Blood 76, 31-35; mEPOR: D'Andrea, AD. *Et al*. (1989) Cell 57, 277-285; hG-CSFR: Fukunaga, R. *et al*. (1990) Proc. Natl. Acad. Sci. USA. 87, 8702-8706; mG-CSFR: Fukunaga, R. *et al*. (1990) Cell 61, 341-350; hTPOR: Vigon, I. *et al*. (1992) 89, 5640-5644; mTPOR: Skoda, RC. *Et al*. (1993) 12, 2645-2653; hInsR: Ullrich, A. *et al*. (1985) Nature 313, 756-761; hFlt-3: Small, D. *et al*. (1994) Proc. Natl. Acad. Sci. USA. 91, 459-463; hPDGFR: Gronwald, RGK. *Et al*. (1988) Proc. Natl. acad. Sci. USA. 85, 3435-3439; hIFN α/β R: Uze, G. *et al*. (1990) Cell 60, 225-234, and Novick, D. *et al*. (1994) Cell 77, 391-400).

In one embodiment, the mutant receptors of the present invention comprise receptors associated with disease onset. The phrase "mutant receptors associated with disease onset" means that the loss of responsiveness to a natural ligand is part of the reason that disease onset is triggered. In the present invention, a mutant receptor is not necessarily the sole factor triggering disease onset, and may be a contributing factor. Many reports have been previously published describing the association of mutant receptors with disease onset; however, in addition to previously reported associations, associations of mutant receptors and disease onset can also be identified by methods of statistical analysis (for example, correlation analyses). Correlation analyses, also called "case control studies", are well known to those skilled in the art (for example, Nishimura, Y, 1991, "Statistical analysis of polymorphisms", Saishin Igaku, 46:909-923; Oka, A. *et al*., 1990, Hum. Mol. Genetics 8, 2165-2170; Ota, M. *et al*., 1999, Am. J. Hum. Genet. 64, 1406-1410; Ozawa, A. *et al*., 1999, Tissue Antigens 53, 263-268). For example, the correlation between a mutant receptor and a disease can be studied by determining the frequency of the mutant receptor in patients and in healthy subjects, and examining whether the patient population has a higher mutant receptor frequency. Typically, differences in the frequency are evaluated using the χ-test. χ is obtained by the equation χ² = Σ(observed value - expected value)²/expected value. A p value is obtained from the χ² value thus determined. Whether a mutant receptor correlates with a disease can be determined based on this p value. For example, when p<0.05, a mutant receptor is considered to correlate with a disease.

There are many reports on mutant receptors known to be involved in disease onset. Such mutant receptors specifically include: mutant thrombopoietin (TPO) receptors, mutant insulin receptors, mutant erythropoietin receptors, mutant growth hormone receptors, mutant common y chain receptors (common receptor of IL-2, IL-4, IL-7, IL-15, and IL-21), mutant androgen receptors (Glutamine Repeats and Neurodegenerative Disease: Molecular Aspects (2001), 261-267, Oxford University press), mutant receptors for proopiomelanocortin (POMC) and melanocortin (Journal of Clinical Endocrinology and Metabolism (2001), 86(4), 1442-1446), mutant ryanodine receptors (Human Mutation (2000), 15(5), 410-417), mutant thyroid-stimulation hormone receptors (Trends in Endocrinology and Metabolism (1998), 9(4), 133-140), and mutant thyrotropin receptors (European Journal of Medical Research (1996), 1(10), 460-464). In the present invention, particularly preferred mutant receptors are mutant thrombopoietin receptors.

Herein, a "natural ligand" refers to a ligand in the body, and is preferably a ligand with the most influence on non-mutant receptor signaling. Normally, the natural ligands of the present invention do not comprise antibodies.

"Agonistic activity" refers to the activity of inducing a change in a certain physiological activity, caused by transducing signals into cells upon the binding of a ligand to a receptor. The physiological activity includes, but is not limited to, growth-promoting activity, survival activity, differentiation activity, transcription activity, membrane transport activity, binding activity, proteolytic activity, phosphorylation/dephosphorylation activity, oxidation/reduction activity, transfer activity, nucleolytic activity, and dehydration activity.

In the present invention, any detection indicator may be used to assay physiological activities, as long as it can measure quantitative and/or qualitative change. For example, it is possible to use cell-free assay indicators, cell-based assay indicators, tissue-based assay indicators, and *in vivo* assay indicators. Indicators that can be used in cell-free assays include enzymatic reactions and quantitative and/or qualitative changes in proteins, DNAs, or RNAs. Such enzymatic reactions include amino acid transfers, sugar transfers, dehydrations, dehydrogenations, and substrate cleavages. Alternatively, the followings can be used: protein phosphorylations, dephosphorylations, dimerizations, multimerizations, catabolisations, dissociations and such; and DNA or RNA amplifications, cleavages, and extensions. For example, protein phosphorylation downstream of a signal transduction pathway may be used as a detection indicator. As indicators in cell-based assays, changes to cell phenotype, for example, quantitative and/or qualitative changes in products, changes in proliferation activity, morphological changes, or changes in cellular properties can be used. Products include secretory proteins, surface antigens, intracellular proteins, and mRNAs. Morphological changes include changes in process formation and/or process number, changes in cell flatness, changes in the degree of elongation/horizontal to vertical ratio, changes in cell size, changes in intracellular structure, heterogeneity/homogeneity of cell populations, and changes in cell density. Such morphological alterations can be observed under a microscope. Cellular properties to be used include anchorage dependency, cytokine-dependent responsiveness, hormone dependency, drug resistance, cell motility, cell migration activity, pulsatility, and alteration in intracellular substances. Cell motility includes cell infiltration activity and cell migration activity. Changes in intracellular substances include changes in enzyme activity, mRNA levels, levels of intracellular signaling molecules such as Ca²⁺ and cAMP, and intracellular protein levels. Furthermore, changes in the cell proliferation activity induced by receptor stimulation can be used as an indicator. The indicators to be used in tissue-based assays include functional changes for the tissue in use. Indicators that can be used for *in vivo* assays include changes in tissue weight, changes in the blood system (for example, changes in the number of hemocytes, protein contents, or enzyme activities), changes in electrolyte levels, and changes in the circulatory system (for example, changes in blood pressure or heart rate).

The methods for measuring such detection indicators are not particularly limited. For example, luminescence, color development, fluorescence, radioactivity, fluorescence polarization values, surface plasmon resonance signals, time-resolved fluorescence, mass, absorption spectrums, light scattering, and fluorescence resonance energy transfers may be used. These measurement methods are known to those skilled in the art and may be appropriately selected depending on the purpose. For example, absorption spectra can be measured using a conventional photometer, plate reader, or such; luminescence can be measured with a luminometer or such; and fluorescence can be measured with a fluorometer or such. Mass can be determined with a mass spectrometer. Radioactivity can be determined with a measurement device such as a gamma counter, depending on the type of radiation. Fluorescence polarization values can be measured using BEACON (TaKaRa). Surface plasmon resonance signals can be obtained using BIACORE. Time-resolved fluorescence, fluorescence resonance energy transfer, or such can be measured with ARVO or such. Furthermore, a flow cytometer can also be used for measuring. In the present invention, it is possible to use a chimeric receptor comprising an extracellular domain of a mutant receptor and a cytoplasmic domain of another protein. For example, when the cytoplasmic domain of G-CSF receptor, EPO receptor, EGF receptor, or thrombopoietin receptor is used, the cell proliferation activity induced by stimulating the receptor can be used as a detection indicator. In assays using cell proliferation activity as a detection indicator, cell lines that die in the absence of ligands are preferably used to improve detection sensitivity. Cytokine-dependent cell lines are particularly preferred because they can be easily passaged. For example, it is possible to use CTLL-2 cells, which are an IL-2-dependent cell line, and 32D cells, FDC-P1 cells, and Ba/F3 cells, which are IL-3-dependent cell lines. These cell lines will die two or three day after the start of culture when a cytokine such as IL-2 or IL-3 that is essential for cell proliferation, is eliminated from the culture media. It is preferable to use FDC-P1 cells and Ba/F3 cells that express a chimeric receptor comprising the cytoplasmic domain of mouse G-CSF receptor.

The ligands of the present invention that have agonistic activity are not particularly limited, as long as they have agonistic activity to mutant receptors. Such ligands may have agonistic activity to both mutant and non-mutant receptors, or may only have agonistic activity to mutant receptors. When the ligands have agonistic activity to both mutant and non-mutant receptors, they may have greater agonistic activity to the non-mutant receptor, or greater agonistic activity to the mutant receptor. Alternatively, the ligands may have comparable agonistic activity to the non-mutant and mutant receptors. Nonetheless, when the chief purpose is to treat a disease caused by a mutant receptor, it is preferable to use a ligand with greater agonistic activity to the mutant receptor than the non-mutant receptor.

The antibodies of the present invention are not particularly limited, as long as they have agonistic activity, and mouse antibodies, rat antibodies, rabbit antibodies, sheep antibodies, camel antibodies, chimeric antibodies, humanized antibodies, human antibodies, and such can be appropriately used. Such antibodies may be polyclonal or monoclonal antibodies. Monoclonal antibodies are preferred because they can be stably produced as homogeneous antibodies. Both polyclonal and monoclonal antibodies can be prepared by methods known to those skilled in the art.

Hybridomas producing monoclonal antibodies can basically be prepared by the conventional methods described below. Specifically, immunization is carried out by a conventional immunization method, using a desired antigen or cells expressing the desired antigen as a sensitizing antigen. The prepared immunocytes are fused with known parental cells by a conventional cell fusion method. The fused cells are screened for monoclonal antibody-producing cells (hybridomas) by conventional screening methods.

The type of sensitizing antigen to be used is not limited. For example, a full-length protein of a receptor of interest, or a partial peptide thereof (for example, an extracellular domain) can be used. The antigens can be prepared by methods known to those skilled in the art; for example, according to methods using baculovirus (for example, see WO 98/46777). Hybridomas can be prepared, for example, by the method of Milstein *et al*. (Kohler, G. and Milstein, C., Methods Enzymol., 1981, 73, 3-46.). When an antigen has weak immunogenicity, the antigen may be conjugated with a large immunogenic molecule such as albumin, to achieve immunization. In addition, the present invention may use recombinant antibodies, produced by gene engineering. The genes encoding the antibodies are cloned from hybridomas, inserted into an appropriate vector, and then introduced into a host (see, e.g., Carl, A. K. Borrebaeck, James, W. Larrick, Therapeutic Monoclonal Antibodies, Published in the United Kingdom by Macmillan Publishers Ltd, 1990). Specifically, using a reverse transcriptase, cDNAs encoding the variable regions (V regions) of the antibodies are synthesized from the mRNAs of the hybridomas. DNAs encoding the variable regions of the antibodies of interest are obtained, and ligated with DNAs encoding desired constant regions (C regions) of the antibodies, and these constructs are inserted into expression vectors. Alternatively, DNAs encoding the variable regions of the antibodies may be inserted into expression vectors comprising the DNAs of the antibody C regions. Those cDNAs are inserted into expression vectors such that the genes are expressed under the regulation of an expression regulatory region, for example, an enhancer and promoter. Host cells are then transformed using the expression vectors, and the antibodies can be expressed. The epitopes on the molecules that are recognized by antibodies of the present invention are not limited to particular epitopes. The antibodies may recognize any epitope on the molecules. Typically, the antibodies recognize epitopes in the extracellular domain. In the present invention, recombinant antibodies artificially modified to reduce heterologous antigenicity against humans can be used. Examples of such recombinant antibodies include chimeric antibodies and humanized antibodies. These modified antibodies can be produced using known methods. A chimeric antibody comprises heavy chain and light chain variable regions of an antibody from a nonhuman mammal such as a mouse, and heavy chain and light chain constant regions of a human antibody. Such an antibody can be obtained by (1) ligating a DNA encoding a variable region of a mouse antibody to a DNA encoding a constant region of a human antibody; (2) inserting the resulting construct into an expression vector; and (3) introducing the vector into a host for production of the antibody. A humanized antibody, which is also called a reshaped human antibody, is obtained by transferring a complementarity determining region (CDR) of an antibody of a nonhuman mammal such as a mouse, to the CDR of a human antibody. Conventional genetic recombination techniques for the preparation of such antibodies are known. Specifically, a DNA sequence designed to ligate a CDR of a mouse antibody with the framework regions (FRs) of a human antibody is synthesized by PCR, using several oligonucleotides constructed to comprise overlapping portions at their ends. A humanized antibody can be obtained by (1) ligating the obtained DNA to a DNA that encodes a human antibody constant region; (2) inserting the resulting construct into an expression vector; and (3) introducing the vector into a host to produce the antibody (see European Patent Application No. EP 239,400, and International Patent Application No. WO 96/02576). Human antibody FRs ligated via the CDR are selected where the CDR forms a favorable antigen-binding site. As necessary, amino acids in the framework region of an antibody variable region may be substituted such that the CDR of a reshaped human antibody forms an appropriate antigen-binding site (Sato, K. *et al*., Cancer Res. (1993) 53, 851-856). Methods for obtaining human antibodies are also known. For example, desired human antibodies with antigen-binding activity can be obtained by (1) sensitizing human lymphocytes with antigens of interest or cells expressing antigens of interest *in vitro*; and (2) fusing the sensitized lymphocytes with human myeloma cells such as U266 (see Japanese Patent Application Kokoku Publication No. (JP-B) H01-59878 (examined, approved Japanese patent application published for opposition)). Alternatively, the desired human antibodies can also be obtained by using antigens of interest to immunize transgenic (Tg) animals comprising a partial or entire repertoire of human antibody genes (see International Patent Application WO 93/12227, WO 92/03918, WO 94/02602, WO 94/25585, WO 96/34096, and WO 96/33735). Furthermore, techniques to obtain human antibodies by panning with a human antibody library are known. For example, the variable regions of human antibodies are expressed as single chain antibodies (scFvs) on the surface of phages, using a phage display method, and the phages that bind to the antigen can be selected. By analyzing the genes of selected phages, the DNA sequences encoding the variable regions of human antibodies that bind to the antigen can be determined. If the DNA sequences of scFvs that bind to the antigen are identified, appropriate expression vectors comprising these sequences can be constructed to obtain human antibodies. Such methods are already well known (see WO 92/01047, WO 92/20791, WO 93/06213, WO 93/11236, WO 93/19172, WO 95/01438, and WO 95/15388). When the antibody genes are isolated and introduced into appropriate hosts to produce antibodies, hosts and expression vectors can be used in appropriate combinations. Eukaryotic host cells that can be used are animal cells, plant cells, and fungal cells. The animal cells include: (1) mammalian cells such as CHO, COS, myeloma, baby hamster kidney (BHK), HeLa, and Vero cells; (2) amphibian cells such as *Xenopus* oocytes; or (3) insect cells such as sf9, sf21, and Tn5. Known plant cells include cells derived from the *Nicotiana* genus such as *Nicotiana tabacum*, which can be callus-cultured. Known fungal cells include yeasts such as the *Saccharomyces* genus, for example *Saccharomyces cerevisiae,* and filamentous fungi such as the *Aspergillus* genus, for example *Aspergillus niger.* Prokaryotic cells can also be used in production systems that utilize bacterial cells. Known bacterial cells include E. *coli* and *Bacillus subtilis*. The antibodies can be obtained by introducing the antibody genes of interest into these cells by transformation, and then culturing the transformed cells *in vitro*.

The antibodies may be minibodies or modified products of antibodies, as long as they can bind to antigens. In the present invention, a minibody comprises an antibody fragment obtained by deleting a portion from a whole antibody (for example, whole IgG). There is no limitation on the type of minibody, as long as it has the ability to bind to an antigen. The antibody fragments of the present invention are not particularly limited, as long as they are portions of whole antibodies. The antibody fragments preferably comprise a heavy chain variable region (VH) or a light chain variable region (VL), and particularly preferably comprise both a VH and VL. Specifically, the antibody fragments include Fab, Fab', F(ab')₂, Fv, and scFv (single-chain Fv). A preferred antibody fragment is scFv (Huston, J. S. *et al*., Proc. Natl. Acad. Sci. U.S.A. (1988) 85, 5879-5883; and Plickthun "The Pharmacology of Monoclonal Antibodies" Vol. 113, Eds. Resenburg and Moore, Springer Verlag, New York, pp. 269-315, (1994)). Such an antibody fragment can be prepared by treating an antibody with an enzyme (for example, papain or pepsin) or by inserting a gene construct encoding the antibody fragment into an expression vector and expressing it in appropriate host cells (see, for example, Co, M. S. *et al*., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. *et al*., Methods Enzymol. (1986) 121, 663-669; and Bird, R. E. and Walker, B. W., Trends Biotechnol. (1991) 9, 132-137). The minibodies of the present invention preferably have a smaller molecular weight than whole antibodies. However, the minibodies may form multimers (for example, dimers, trimers, or tetramers), and thus their molecular weights can be greater than those of whole antibodies.

Preferred minibodies of the present invention comprise two or more antibody VHs and two or more antibody VLs, in which each of the variable regions are directly linked, or indirectly linked together via linkers or such. The linkages may be covalent or non-covalent bonds, or comprise both covalent and non-covalent bonds. More preferred minibodies are antibodies comprising two or more VH-VL pairs formed via non-covalent bonding between VH and VL. The distance between the two VH-VL pairs in a minibody is preferably less than that in the whole antibody.

Particularly preferred minibodies of the present invention are diabodies and sc(Fv)2. A diabody is a dimerized fragment in which two variable regions are linked together via a linker or such (for example, scFv) (hereinafter, referred to as "a fragment constituting a diabody"), and which typically comprises two VL and two VH (P. Holliger *et al*., Proc. Natl. Acad. Sci. USA, 90, 6444-6448 (1993), EP 404097; WO 93/11161; Johnson *et al*., Methods in Enzymology, 203, 88-98, (1991); Holliger *et al*., Protein Engineering, 9, 299-305, (1996); Perisic *et al*., Structure, 2, 1217-1226, (1994); John *et al*., Protein Engineering, 12(7), 597-604, (1999); Holliger *et al*., Proc. Natl. Acad. Sci. USA. 90, 6444-6448, (1993); Atwell *et al*., Mol. Immunol. 33, 1301-1312, (1996)). The links between fragments constituting a diabody may be non-covalent or covalent bonds, and are preferably non-covalent bonds.

Alternatively, two fragments constituting a diabody can be linked together via a linker to form a single-chain diabody (scDiabody). When the fragments constituting the diabody are linked together using a long linker, comprising approximately 20 amino acids, it is possible to link the fragments constituting the diabody in the same chain using a non-covalent bond, forming a dimer.

Fragments constituting diabodies include a VL and VH linked together, a VL and VL linked together, a VH and VH linked together, and the like. A VH and VL linked together is preferred. There is no limitation on the type of linker for linking a variable region and variable region in a fragment constituting a diabody. However, it is preferable to use a linker short enough to prevent formation of a non-covalent bond between variable regions in the same fragment. Those skilled in the art can appropriately select the length of such linkers; however, their length is typically 2 to 14 amino acids, preferably 3 to 9 amino acids, and particularly preferably 4 to 6 amino acids. In these cases, the linker between the VL and VH encoded by the same fragment is short, and thus no non-covalent bonds are formed between VL and VH on the same chain. Thus, a single-chain V region fragment is not formed, and the VL and VH form dimers with other fragments via non-covalent bonds. Further, based on the same principle for producing diabodies, a multimerized antibody such as a trimer or tetramer can be prepared by linking three or more fragments constituting a diabody.

The sc(Fv)2 of the present invention are single-chain minibodies produced by linking two VHs and two VLs with linkers and such (Hudson *et al*., 1999, J Immunol. Methods 231:177-189). sc(Fv)2 exhibit a particularly high agonistic activity compared to whole antibodies and other minibodies. sc(Fv)2 can be produced, for example, by linking scFv molecules with a linker.

In a preferable antibody, two VHs and two VLs are arranged in the order of VH, VL, VH, and VL ([VH]-linker-[VL]-linker-[VH]-linker-[VL]), beginning from the N terminus of a single-chain polypeptide.

The order of the two VHs and two VLs is not limited to the above arrangement, and they may be arranged in any order. Examples of arrangements are listed below:
[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL]-linker-[VL]-linker-[VH]-linker-[VH]
[VL]-linker-[VH]-linker-[VL]-linker-[VH]

The linkers for linking the variable regions of an antibody can be arbitrary peptide linkers that can be introduced by genetic engineering, or synthetic linkers (for example, see Protein Engineering, 9(3), 299-305, 1996). However, peptide linkers are preferred in the present invention. There are no limitations as to the length of the peptide linkers. The length can be appropriately selected by those skilled in the art, depending on the purpose, and is typically 1 to 100 amino acids, preferably 3 to 50 amino acids, more preferably 5 to 30 amino acids, and even more preferably 12 to 18 amino acids (for example, 15 amino acids).

For example, such peptide linkers include: 
Ser
Gly Ser
Gly Gly Ser
Ser Gly Gly
Gly Gly Gly Ser
Ser Gly Gly Gly
Gly Gly Gly Gly Ser
Ser Gly Gly Gly Gly
 
Gly Gly Gly Gly Gly Ser
Ser Gly Gly Gly Gly Gly
Gly Gly Gly Gly Gly Gly Ser
Ser Gly Gly Gly Gly Gly Gly
(Gly Gly Gly Gly Ser)n
(Ser Gly Gly Gly Gly)n
where n is an integer of one or larger. The lengths and sequences of peptide linkers can be appropriately selected by those skilled in the art, depending on the purpose.

In an embodiment of the present invention, particularly preferable sc(Fv)2 include the sc(Fv)2 below:
[VH]-peptide linker (15 amino acids)-[VL]-peptide linker (15 amino acids)-[VH]-peptide linker (15 amino acids)-[VL]

Synthetic linkers (chemical cross-linking agents) include cross-linking agents routinely used to cross-link peptides; for example, N-hydroxy succinimide (NHS), disuccinimidyl suberate (DSS), bis(succinimidyl) suberate (BS³), dithiobis(succinimidyl propionate) (DSP), dithiobis(succinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl] sulfone (BSOCOES), and bis[2-(succinimidoxycarbonyloxy)ethyl] sulfone (sulfo-BSOCOES). These cross-linking agents are commercially available.

In general, three linkers are required to link four antibody variable regions together. The linkers to be used may be of the same or different types. In the present invention, a preferable minibody is a diabody, even more preferably, an sc(Fv)2. Such a minibody can be prepared by treating an antibody with an enzyme, for example, papain or pepsin, to generate antibody fragments, or by constructing DNAs encoding those antibody fragments and introducing them into expression vectors, followed by expression in an appropriate host cell (see, for example, Co, M. S. *et al*., 1994, J. Immunol. 152, 2968-2976; Better, M. and Horwitz, A. H., 1989, Methods Enzymol. 178, 476-496; Pluckthun, A. and Skerra, A., 1989, Methods Enzymol. 178, 497-515; Lamoyi, E., 1986, Methods Enzymol. 121, 652-663; Rousseaux, J. *et al*., 1986, Methods Enzymol. 121, 663-669; Bird, R. E. and Walker, B. W., 1991, Trends Biotechnol. 9, 132-137).

Antibodies with extremely high agonistic activity can be prepared by converting whole antibodies into minibodies.

Modified antibodies for use include antibodies linked to various molecules, such as polyethylene glycol (PEG). Alternatively, it is also possible to link an antibody to a radioisotope, chemotherapeutic agent, or cytotoxic substance such as a bacterial toxin. Such modified antibodies can be prepared by chemically modifying an obtained antibody. Methods for modifying antibodies have been previously established in the art.

Further, antibodies for use in the present invention may be bispecific antibodies. A bispecific antibody may comprise two antigen-binding sites that each recognizes different epitopes on a certain molecule. Alternatively, one of the antigen-binding sites may recognize a certain molecule, and the other may recognize a radioactive substance, chemotherapeutic agent, or cytotoxic substance such as a cell-derived toxin. When such cytotoxic substances are used, tumor cell growth can be suppressed by directly adding the cytotoxic substance to cells that express a certain molecule, and specifically damaging the tumor cells. The bispecific antibodies can be prepared by linking pairs of H and L chains from two types of antibodies, or by fusing hybridomas which produce different monoclonal antibodies to produce a fused cell producing a bispecific antibody. Further, bispecific antibodies can be prepared using genetic engineering techniques.

Antibodies in which sugar chains have been modified can also be used in the present invention. Techniques for modifying antibody sugar chains have been previously reported (for example, WO 00/61739 and WO 02/31140).

An "antibody" of the present invention includes the antibodies described above.

The antibodies expressed or produced as described above can be purified by conventional protein purification methods. The antibodies can be separated and purified, for example, by the combined use of methods appropriately selected from affinity columns such as a protein A column, column chromatography, filtration, ultrafiltration, salting out, dialysis, and so on (Antibodies A Laboratory Manual. Ed Harlow, David Lane, Cold Spring Harbor Laboratory, 1988).

The antigen-binding activity of an antibody can be assayed by conventional methods (Antibodies A Laboratory Manual. Ed Harlow, David Lane, Cold Spring Harbor Laboratory, 1988). For example, an enzyme-linked immunosorbent assay (ELISA), enzyme immunoassay (EIA), radioimmunoassay (RIA), or immunofluorescence method can be used.

The present invention also provides methods of screening for ligands having agonistic activity to mutant receptors. In these methods, first, a test substance (test compound) is contacted with a mutant receptor. The "contact" of a test substance with a mutant receptor is typically achieved by adding the test substance to a culture medium or extract of cells expressing the mutant receptor. The methods for achieving contact are not limited to this method. When the test substance is a protein or such, the "contact" can be achieved by introducing a DNA vector expressing the protein into the cells.

In these methods, the next step comprises detecting signals from the mutant receptor. Signals can be detected by the methods described above.

Next, ligands with agonistic activity are selected based on comparison with cases when a test substance (control) is not contacted. The ligands selected in this way are expected to become therapeutic agents for treating or preventing diseases associated with receptor deficiencies or mutations.

In another embodiment, a screening method of the present invention comprises the first step of contacting a test substance with a normal receptor and measuring agonistic activity. Next, the same test substance is contacted with a mutant receptor, and agonistic activity is measured. Ligands with high agonistic activity to the mutant receptor as compared to a normal receptor are then selected.

In still another embodiment, a screening method of the present invention comprises the first step of contacting a test substance with a normal receptor and measuring the agonistic activity. Next, the same test substance is contacted with a mutant receptor and agonistic activity is measured. Ligands with agonistic activity to both the mutant receptor and normal receptor are then selected.

Measuring agonistic activity in the methods described above can be achieved as described above.

Substances (compounds) obtained by the above-described screening methods of the present invention are also comprised in the present invention.

Since the ligands of the present invention (for example, antibodies) have agonistic activity, they are expected to be effective therapeutic agents for diseases caused by the impaired response of receptors on which the ligands act. Such impaired responses are attributed to receptor deficiencies or mutations. Specifically, the present invention provides therapeutic agents comprising the above-described ligands of the present invention, which are used to treat diseases caused by the mutant receptors. Representative examples of the diseases described above are thrombocytopenia, type II diabetes mellitus, and Laron syndrome.

A preferred example of a disease of the present invention is congenital amegakaryocytic thrombocytopenia (CAMT).

When ligands of the present invention or substances (compounds) obtained by the methods for screening of the present invention are used as pharmaceutical compositions, they can be formulated by methods known to those skilled in the art. As necessary, the ligands or substances can be used orally, for example, as sugar-coated tablets, capsules, elixirs, or microcapsules, or parenterally, as injections of sterile solutions or suspensions comprising water or other pharmaceutically acceptable liquids. For example, the ligands or the substances can be formulated by appropriately combining with pharmaceutically acceptable carriers or solvents, specifically, sterile water or physiological saline, vegetable oils, emulsifiers, suspending agents, surfactants, stabilizers, flavoring agents, excipients, vehicles, preservatives, binding agents, and such, and mixing at a unit dosage and form required by accepted pharmaceutical implementations. In such formulations, the amount of the active ingredient should be within the required range.

Additives in the tablets or capsules can include, for example, binders such as gelatin, corn starch, gum tragacanth, and gum Arabic; excipients such as crystalline cellulose; swelling agents such as corn starch, gelatin, and alginic acid; lubricants such as magnesium stearate; edulcorants such as sucrose, lactose, or saccharin; and flavoring agents such as peppermint, Gaultheria adenothrix oil, and cherry. When the unit dosage form is a capsule, the above-described materials can also comprise a liquid carrier such as oil. A sterile composition for injection can be formulated using a vehicle such as distilled water used for injection, according to standard protocols.

Aqueous solutions used for injections include physiological saline and isotonic solutions comprising glucose or other adjunctive agents such as D-sorbitol, D-mannose, D-mannitol, and sodium chloride. They may also be combined with an appropriate solubilizing agent such as alcohol, specifically, ethanol, polyalcohol such as propylene glycol or polyethylene glycol, or non-ionic detergent such as polysorbate 80™ or HCO-50.

Oil solutions include sesame oils and soybean oils, and can be combined with solubilizing agents such as benzyl benzoate or benzyl alcohol. They may also be formulated with buffers, for example, phosphate buffers or sodium acetate buffers; analgesics, for example, procaine hydrochloride; stabilizers, for example, benzyl alcohol or phenol; or anti-oxidants. The prepared injections are typically aliquoted into appropriate ampules.

The administration may be carried out orally or parenterally, and preferably parenterally. Specifically, injection, intranasal administration, intrapulmonary administration, percutaneous administration, or such can be used. Injections include intravenous injections, intramuscular injections, intraperitoneal injections, and subcutaneous injections. The injection solutions can also be systemically or locally administered. The administration methods can be properly selected according to the patient's age, condition, and such. When the compounds can be encoded by DNA, the DNA can be inserted into a vector for gene therapy, and gene therapy can be carried out. The dosage may be, for example, in the range of 0.0001 to 1,000 mg/kg body weight. Alternatively, the dosage may be, for example, in the range of 0.001 to 100,000 mg/person. However, the dosage is not restricted to the values described above. The dosage and administration methods depend on a patient's weight, age, and condition, and can be appropriately selected by those skilled in the art.

### Brief Description of the Drawings

Fig. 1 is a diagram showing the structure of a pCOS2-hMPLfull vector.
Fig. 2 is a diagram showing the structure of a pCOS2-hMPLfullG305C vector.
Fig. 3 is a diagram showing the structure of a pBACsurf1-hMPL-FLAG vector.
Fig. 4 is a diagram showing the agonistic activity of each of the diabodies and hTPO in pCOS2-HA-Ba/F3. The vertical axis indicates O.D.450/655 nm and the horizontal axis indicates the concentration.

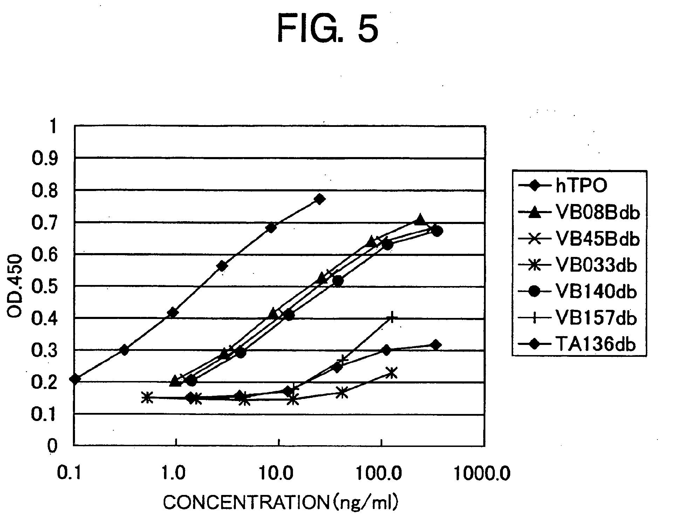

Fig. 5 is a diagram showing the agonistic activity of each of the diabodies and hTPO in hMPL-Ba/F3. The vertical axis indicates O.D.450/655 nm and the horizontal axis indicates the concentration.

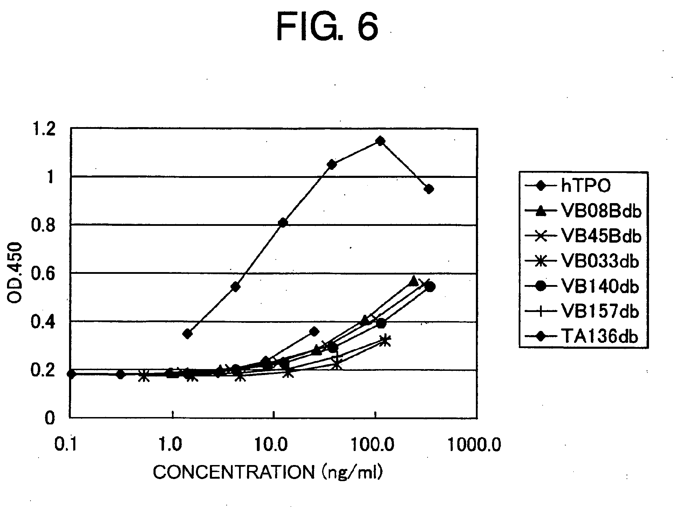

Fig. 6 is a diagram showing the agonistic activity of each of the diabodies and hTPO in hMPL(G305C)-Ba/F3. The vertical axis indicates O.D.450/655 nm and the horizontal axis indicates the concentration.
Fig. 7 is a diagram showing the structure of a pCOS2-hMPLfullC769T vector.
Fig. 8 is a diagram showing the structure of a pCOS2-hMPLfullC823A vector.
Fig. 9 is diagram showing the construction of a TA136 sc(Fv)2 gene.
Fig. 10 is a diagram showing the structure of a pCXND3-TA136 sc(Fv)2 vector.
Fig. 11 is a diagram showing the agonistic activity of TA136 db and TA136 sc(Fv)2 in hMPL-Ba/F3 cells. The vertical axis indicates O.D.450/655 nm and the horizontal axis indicates the concentration.
Fig. 12 is a diagram showing the agonistic activity of TA136 db and TA136 sc(Fv)2 in hMPL(G305C)-Ba/F3 cells. The vertical axis indicates O.D.450/655 nm and the horizontal axis indicates the concentration.
Fig. 13 is a diagram showing the agonistic activity of TA136 db and TA136 sc(Fv)2 in hMPL(C769T)-Ba/F3 cells. The vertical axis indicates O.D.450/655 nm and the horizontal axis indicates the concentration.
Fig. 14 is a diagram showing the agonistic activity of TA136 db and TA136 sc(Fv)2 in hMPL(C823A)-Ba/F3 cells. The vertical axis indicates O.D.450/655 nm and the horizontal axis indicates the concentration.

### Best Mode for Carrying Out the Invention

Herein below, the present invention will be specifically described using Examples, however, it is not to be construed as being limited thereto.

### [Example 1] Establishment of a Ba/F3 cell line

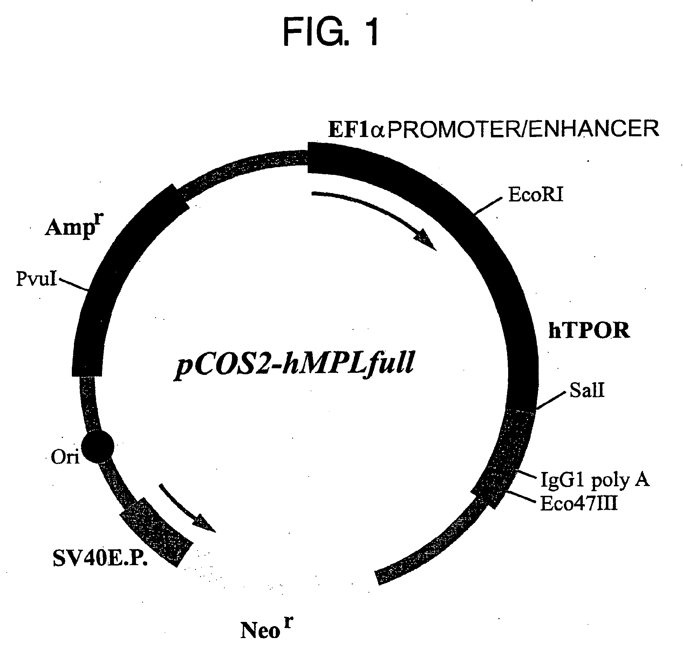

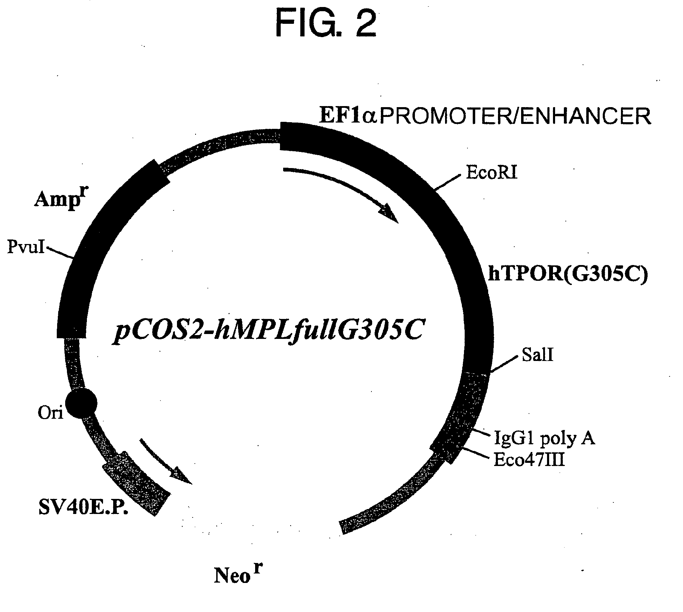

Several reports have described CAMT patients carrying the G305C (R102P) mutation in their thrombopoietin receptor gene. In this context, an expression vector for the thrombopoietin receptor gene carrying the G305C (R102P) mutation was constructed and introduced into Ba/F3 cells by the method described below. The prepared DNA fragments were: the normal thrombopoietin receptor gene (SEQ ID NO: 1) and the mutant gene in which the C at nucleotide position 305 from the initiation codon has been substituted for G (SEQ ID NO: 3). These DNA fragments were digested with the restriction enzymes *EcoRI* and *Sal*I, and introduced into the *Eco*RI*-Sal*I site of the animal cell expression vector pCOS2-Ha to prepare pCOS2-hMPLfull (Fig. 1) and pCOS2-hMPLfullG305C (Fig. 2).

After the plasmids pCOS2-hMPLfull, pCOS2-hMPLfullG305C, and as a negative control pCOS2-Ha were treated with *Pvu*I, 20 µg of each plasmid was transfected into Ba/F3 cells under the conditions described below. The gene was introduced at a cell density of 1x 10⁷ cells/ml in PBS using GENE PULSER II (BIO-RAD) (Gene Pulser Cuvette, 0.4 cm; 0.33 kV; 950 µF). The medium was then changed with RPMI1640 comprising 10% FBS, 1 ng/ml rmIL3 (Pepro tech), 500 µg/ml Geneticin(GIBCO), 100 unit/ml penicillin, and 100 µg/ml streptomycin to select cells. As a result, hMPL-Ba/F3, hMPL(G305C)-Ba/F3, and pCOS2-HA-Ba/F3 cell lines were obtained from the respective vectors described above.

### [Example 2] Preparation of the extracellular domain protein of thrombopoietin receptor

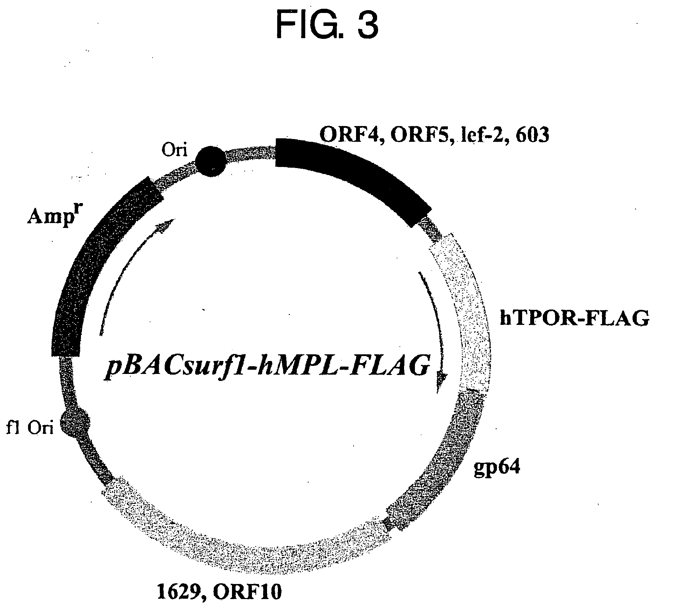

To prepare the antigen for producing anti-thrombopoietin receptor antibodies, a system for producing and secreting the extracellular domain of human thrombopoietin receptor using the insect cell line Sf9 was constructed as described below. A gene construct comprising FLAG tag placed downstream of the extracellular domain of human thrombopoietin receptor (Gln26-Trp491) was prepared and inserted into the *Pst*I*-Sma*I site of pBACsurf-1 (Novagen), to construct pBACsurf1-hMPL-FLAG (Fig. 3). The resulting gene construct (SEQ ID NO: 5) can secrete the extracellular domain of thrombopoietin receptor using a secretory signal sequence derived from baculovirus gp64 protein. 4 µg of the vector was transfected into Sf9 cells using Bac-N-Blue Transfection Kit (Invitrogen), according to the protocol attached to the kit. After three days of culture, the culture supernatants were collected and recombinant viruses were isolated using plaque assays. Stock viral solutions were prepared, and then infected to Sf9 cells. The resulting culture supernatants were collected, and adsorbed to a Q Sepharose Fast Flow column (Pharmacia). The column was eluted with PBS comprising 500 mM NaCl and 0.01% Tween20. The eluate was adsorbed to M2 Affinity Resin (Sigma). The resins were eluted with 100 mM Glycine-HCl (pH 3.5) comprising 0.01% Tween20. Immediately after elution, the eluate was neutralized with 1M Tris-Cl (pH 8.0). The resulting solution was treated by gel filtration chromatography using Superdex 200 26/60 (PBS comprising 0.01% Tween20) to purify the protein.

### [Example 3] Preparation of anti-thrombopoietin receptor diabody

MRL/lpr mice were immunized seven times with the purified protein of the TPOR extracellular domain. The first immunization was carried out using 100 µg of the protein, and subsequent immunizations were each performed using 50 µg of protein. The immunized cells were fused with P3-X63-Ag8-U1 (P3U1) cells by methods commonly used to prepare hybridomas. The hybridomas that produced anti-thrombopoietin receptor antibodies were selected by ELISA assay using the purified protein (VB08B, VB45B, VB033, VB140, and VB157).

Meanwhile, Balb/c mice were immunized a total of 11 times with hMPL-Ba/F3 cells at one-week to five-month intervals. 1.0x 10⁷ cells were intraperitoneally administered to the mice each time. Hybridomas were then prepared by the same method as described above. The hybridomas that produced anti-thrombopoietin receptor antibody were selected (TA136).

The cDNAs for the variable regions of the antibody H and L chains were cloned from each of the hybridomas thus prepared. The cloned cDNAs were sequenced. Based on the nucleotide sequences, genes encoding diabodies were designed with FLAG tag at their C termini (VB08B db, VB45B db, VB033 db, VB140 db, VB157 db, and TA136 db), and inserted into the expression vector pCXND3 for animal cells (pCXND3-VB08B db, pCXND3-VB45B db, pCXND3-VB033 db, pCXND3-VB140 db, pCXND3-VB157 db, pCXND3-TA136 db). Each prepared vector was introduced into COS7 cells, and the culture supernatant was collected after three days of culture. The concentration of diabody in each culture supernatant was determined by BIAcore (Pharmacia) using M2 antibody (Sigma).

### [Example 4] Assay for diabody dependency of Ba/F3 cell line

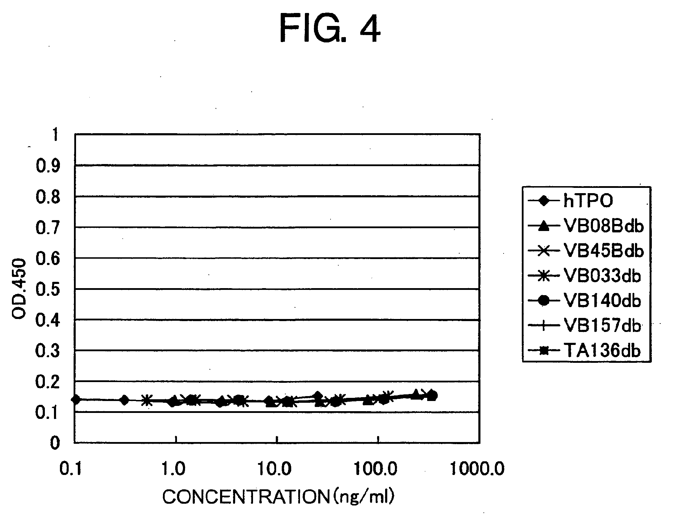

pCOS2-HA-Ba/F3 cells, hMPL_Ba/F3 cells, and hMPL(G305C)-Ba/F3 cells were each diluted to 2.0x 10⁵ cells/ml with medium (RPMI1640 comprising 10% FBS, 100 unit/ml penicillin, and 100 µg/ml streptomycin). The cells were aliquoted (60 µl/well) into the wells of 96-well plates. hTPO (R&D) was diluted to a final concentration of 25 µg/ml with CHO-S-SFM II, and then aliquoted into the wells (40 µl/well). Each diabody/COS7 sup (VB08B db, VB45B db, VB033 db, VB140 db, VB157 db, and TA136 db) was diluted 1, 3, 9, 27, 81, and 243 times using CHO-S-SFM II, and then aliquoted into the wells (40 µl/well). The plates were incubated for 24 hours, and then Cell Count Reagent (nacalai tesque) was added to each well (10 µl/well). The O.D.450/655 nm of each well was measured after two hours of culture. The result showed that the responsiveness of hMPL(G305C)-Ba/F3 cells to hTPO and other agonistic antibodies was markedly decreased. However, TA136 db (SEQ ID NO: 7) was found to exhibit strong agonistic activity to hMPL(G305C)-Ba/F3 cells, while it exhibited weak agonistic activity to hMPL_Ba/F3 cells expressing the normal receptor (Figs. 4to 6).

In SEQ ID NO: 8, the amino acid sequence from positions 49 to 54 corresponds to heavy chain CDR1; from 69 to 84 corresponds to heavy chain CDR2; from 117 to 123 corresponds to heavy chain CDR3; from 163 to 174 corresponds to light chain CDR1; from 190 to 196 corresponds to light chain CDR2; and from 229 to 237 corresponds to light chain CDR3.

### [Example 5] Establishment of a Ba/F3 cell line (2)

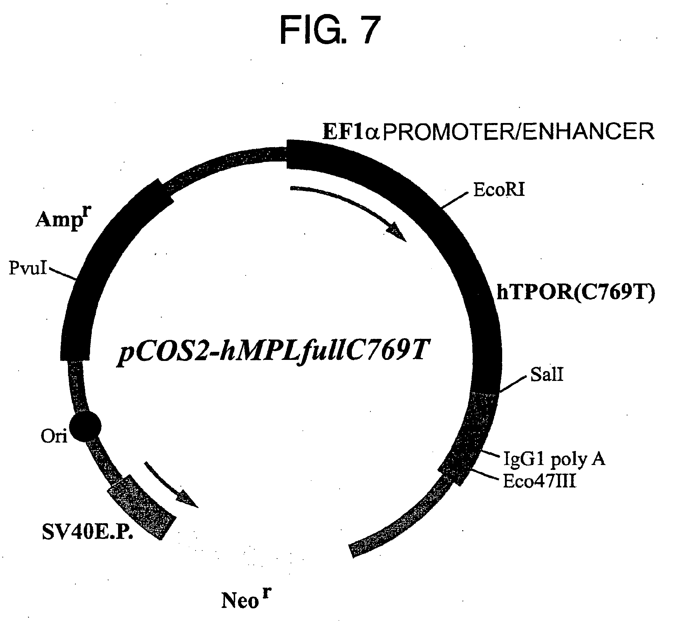

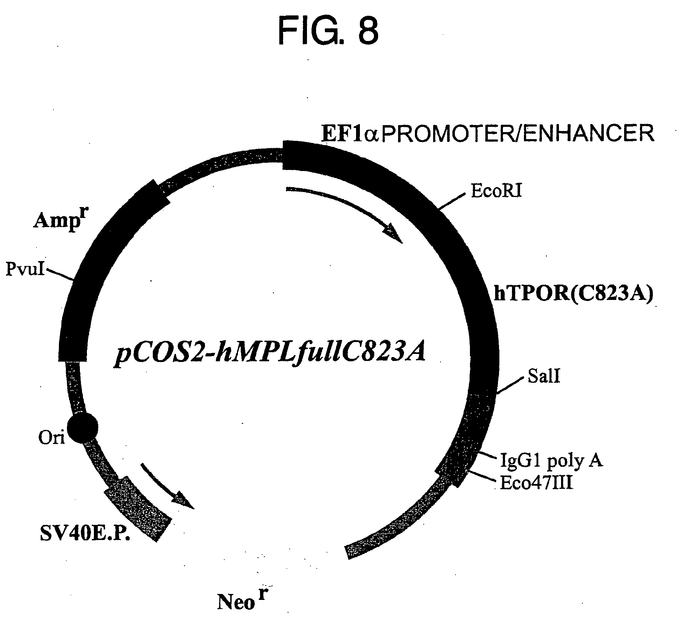

As in Example 1, expression vectors were constructed for each of the mutant thrombopoietin receptor genes carrying the C769T (R257C) mutation and the C823A (P275T) mutation, which were found in some CAMT patients. The constructs were introduced into Ba/F3 cells. Nucleotide T was substituted for the nucleotide C at position 769 from the initiation codon in the thrombopoietin receptor gene (SEQ ID NO: 1), to produce the gene of SEQ ID NO: 9; and nucleotide A was substituted for the nucleotide C at position 823, to produce the gene of SEQ ID NO: 11. These DNA fragments were digested with the restriction enzymes *Eco*RI and *Sal*I, and introduced into the *Eco*RI*-Sal*I site of the animal cell expression vector pCOS2-Ha to obtain pCOS2-hMPLfullC769T (Fig. 7) and pCOS2-hMPLfullC823A (Fig. 8), respectively.

After treating the plasmids pCOS2-hMPLfullC769T and pCOS2-hMPLfullC823A with *Pvu*I, 20 µg of each of them was transfected into Ba/F3 cells under the conditions described below. The gene transfer was carried out by electroporation at a cell density of 1x 10⁷ cells/ml in PBS using GENE PULSER II (BIO-RAD) (Gene Pulser Cuvette 0.4 cm; 0.33 kV; 950 µF). The medium was then changed to RPMI1640 comprising 10% FBS, 1 ng/ml rmIL3 (Pepro tech), 500 µg/ml Geneticin (GIBCO), 100 units/ml penicillin, and 100 µg/ml streptomycin to select cells. As a result, hMPL(C769T)-Ba/F3 and hMPL(C823A)-Ba/F3 cell lines were respectively obtained from the vectors described above.

### [Example 6] Preparation of anti-thrombopoietin receptor antibody sc(FV)2

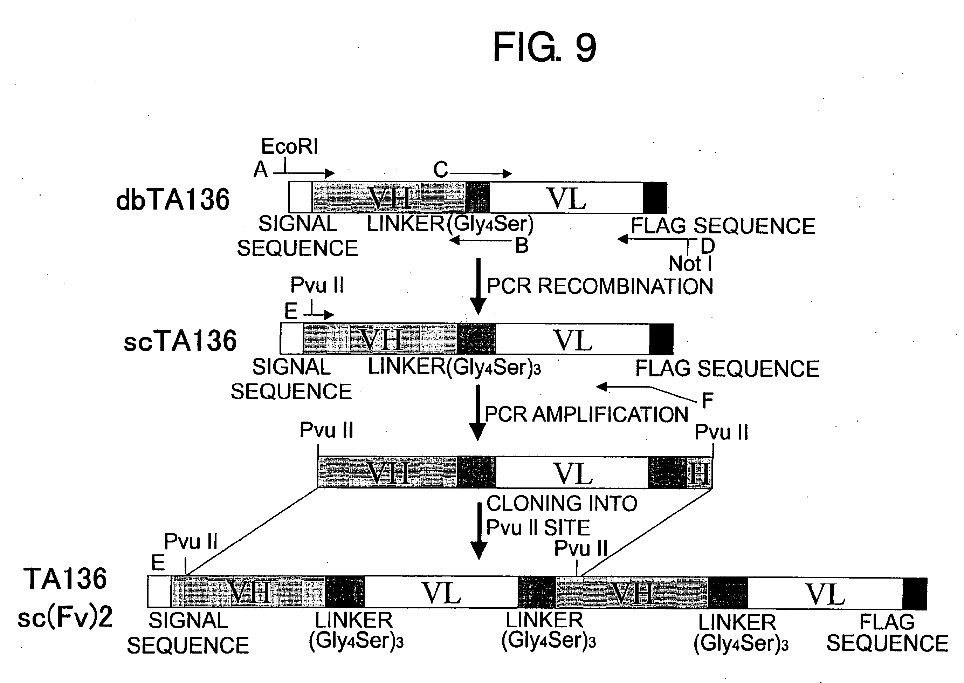

TA136 sc(Fv)2 gene was constructed using pCXND3-TA136 db described above by the procedure described below (Fig. 9).

PCR was carried out using a combination of primer A
(TAGAATTCCACCATGAGAGTGCTGATTCCTTTGTGGCTGTTCACAGCCTTTCCTGGTA CCCTGTCTGATGTGCAGCTGCAGG/SEQ ID NO: 15) and primer B (TGGGTGAGAACAATTTGCGATCCGCCACCACCCGAACCACCACCACCCGAACCACC ACCACCTGAGGAGACGGTGACTGAGG/SEQ ID NO: 16); and also a combination of primer C
(CAGTCACCGTCTCCTCAGGTGGTGGTGGTTCGGGTGGTGGTGGTTCGGGTGGTGGC GGATCGCAAATTGTTCTCACCCAGTC/SEQ ID NO: 17) and primer D (ATTGCGGCCGCTTATCACTTATCGTCGTCATCCTTGTAGTCTTTGATTTCCAGCTTGGT G/SEQ ID NO: 18). The resulting PCR products were combined and used as a template in another PCR using primers A and D. The resulting DNA fragment of about 800 bp was digested using the restriction enzymes *EcoR*I and *Not*I, and cloned into pBacPAK9 (CLONTECH) to prepare pBacPAK9-scTA136.

PCR was then carried out using pBacPAK9-scTA136 as a template with primer E (GATGTGCAGCTGCAGGAGTCGGGAC/SEQ ID NO: 19) and primer F (CCTGCAGCTGCACATCCGATCCACCGCCTCCCGAACCACCACCACCCGATCCACCAC CTCCTTTGATTTCCAGCTTGGTGC/SEQ ID NO: 20). The resulting DNA fragment of approximately 800 bp was cloned into the pGEM-T Easy vector (Promega).

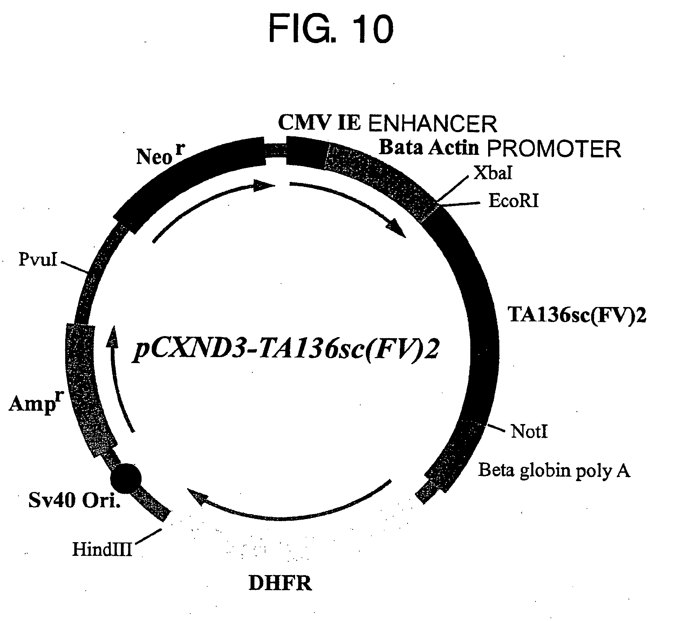

After confirming the nucleotide sequence, the DNA was digested with the restriction enzyme *Pvu*II. The resulting DNA fragment of approximately 800 bp was inserted into the *Pvu*II site of pBacPAK9-scTA136 to prepare pBacPAK9-TA136 sc(Fv)2. The prepared vector was digested with the restriction enzymes *Eco*RI and *Not*I. The resulting DNA fragment of approximately 1600 bp was cloned into the expression vector pCXND3 to prepare pCXND3-TA136 sc(Fv)2 (SEQ ID NO: 13; Fig. 10).

### [Example 7] Evaluation of TPO-like agonistic activities of TA136 db and TA136 sc(Fv)2

The DNA constructs pCXND3-TA136 db and pCXND3-TA136 sc(Fv)2 were introduced into COS7 cells. Their respective culture supernatants were collected after three days of culture. The diabody concentrations in the prepared culture supernatants were determined by BIAcore (Pharmacia) using M2 antibody (Sigma).

hMPL-Ba/F3 cells, hMPL(G305C)-Ba/F3 cells, hMPL(C769T)-Ba/F3 cells, and hMPL(C823A)-Ba/F3 cells were each diluted to 4.0x 10⁵ cells/ml using medium (RPMI1640 comprising 10% FBS, 100 unit/ml penicillin, and 100 µg/ml streptomycin). The cells were aliquoted into the wells of 96 well plates (60 µl/well). 40 µl of hTPO (R&D) and the culture supernatant of the COS7 cells described above were added to each well, and the plate was incubated for 24 hours. 10 µl of Cell Count Reagent (Nacalai Tesque) was added to each well. O.D.450/655 nm was determined after incubating the plate for two hours.

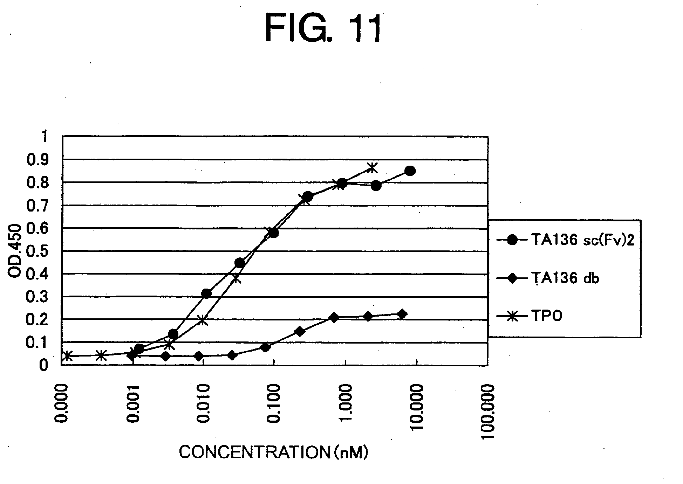

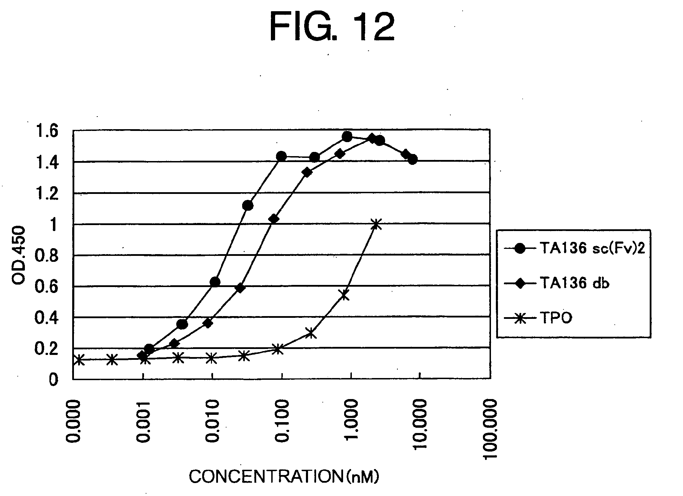

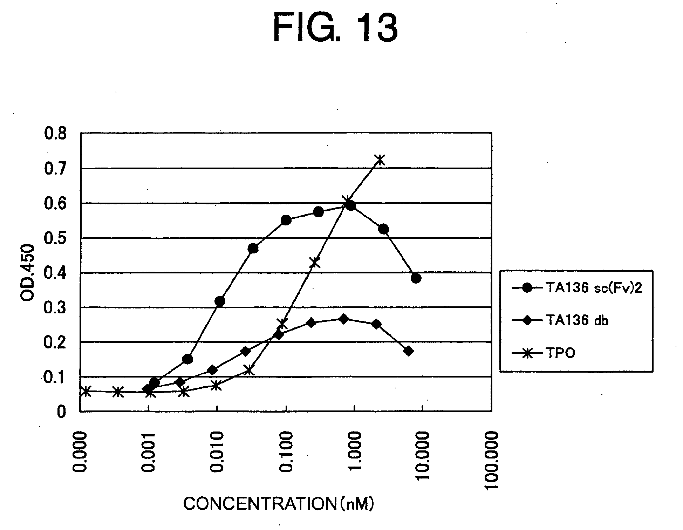

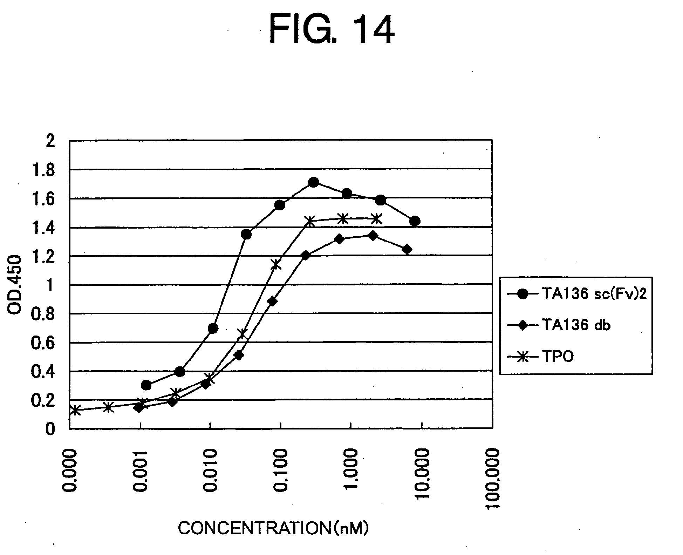

The results showed that, in all three mutant thrombopoietin receptor cell lines, TA136 sc(Fv)2 exhibited much stronger agonistic activity than hTPO and TA136 db (Figs. 12 to 14). Furthermore, TA136 db was found to show agonistic activity comparable to that of the natural ligand hTPO when converted into sc(Fv)2, although in hMPL-Ba/F3 cells expressing normal thrombopoietin receptor, TA136 db exhibited weaker activity than that of hTPO (Fig. 11).

### Industrial Applicability

The present invention provides ligands (antibodies) to treat patients with diseases caused by mutant receptors, for example, CAMT; polynucleotides encoding these antibodies; vectors comprising the polynucleotides; host cells comprising the vectors; and methods for producing the antibodies. In addition, the present invention also provides methods for gene therapy using polynucleotides that encode the antibodies. The methods of the present invention provide methods for treating various genetic diseases caused by mutations in genes that encode cell membrane proteins. Henceforth, individualized genetic diagnosis is likely to become widely available for patients. The antibody engineering techniques of the present invention enable the development of pharmaceutical agents matched to individual genotypes.

## Claims

1. A ligand having agonistic activity to a mutant receptor.

2. The ligand of claim 1, where the ligand is an antibody.

3. The ligand of claim 1 or 2, where the ligand has greater agonistic activity to the mutant receptor than the natural ligand.

4. The ligand of any one of claims 1 to 3, where the mutant receptor is a receptor resulting from a mutation(s) in the amino acid sequence.

5. The ligand of any one of claims 1 to 4, where the mutant receptor has lost responsiveness to the natural ligand.

6. The ligand of any one of claims 1 to 5, where the mutant receptor causes a disease.

7. The ligand of any one of claims 1 to 6, where the mutant receptor is a mutant thrombopoietin receptor.

8. The ligand of claim 2, where the antibody is a minibody.

9. The ligand of claim 8, where the minibody is a diabody.

10. A method for transducing a signal to a mutant receptor by binding a ligand.

11. The method of claim 10, where the ligand is an antibody.

12. The method of claim 10 or 11, where the mutant receptor results from an amino acid mutation(s).

13. The method of any one of claims 10 to 12, where the mutant receptor has lost responsiveness to the natural ligand.

14. The method of any one of claims 10 to 13, where the mutant receptor is associated with disease onset.

15. The method of any one of claims 10 to 14, where the mutant receptor is a mutant thrombopoietin receptor.

16. A method for treating a disease caused by a mutant receptor, by binding a ligand to the mutant receptor.

17. The method of claim 16, where the ligand is an antibody.

18. A method of screening for a ligand having agonistic activity to a mutant receptor, where the method comprises the steps of:
(a) contacting a test substance with the mutant receptor;
(b) detecting a signal in the mutant receptor; and
(c) selecting a ligand having agonistic activity.

19. A method of screening for a ligand having agonistic activity to a mutant receptor, where the method comprises the steps of:
(a) determining agonistic activity to a normal receptor;
(b) determining agonistic activity to the mutant receptor; and
(c) selecting a ligand having greater agonistic activity to the mutant receptor than the normal receptor.

20. A method of screening for a ligand having agonistic activity to a normal and a mutant receptor, where the method comprises the steps of:
(a) determining agonistic activity to the normal receptor;
(b) determining agonistic activity to the mutant receptor; and
(c) selecting a ligand having agonistic activity to both the normal and the mutant receptors.

21. The method of any one of claims 18 to 20, where the ligand is an antibody.

22. A substance obtained by the method of any one of claims 18 to 21.

23. A therapeutic agent for a disease caused by a mutant receptor, where the agent comprises a ligand of the mutant receptor.

24. The therapeutic agent of claim 23, where the ligand is the ligand of any one of claims 1 to 9.

25. The therapeutic agent of claim 23, where the ligand is an antibody.

26. The therapeutic agent of any one of claims 23 to 25, where the mutant receptor results from an amino acid mutation(s).

27. The therapeutic agent of any one of claims 23 to 26, where the mutant receptor has lost responsiveness to the natural ligand.

28. The therapeutic agent of any one of claims 23 to 27, where the mutant receptor is a mutant thrombopoietin receptor.

29. The therapeutic agent of any one of claims 23 to 28, where the disease is congenital amegakaryocytic thrombocytopenia.
